(19) Europäisches Patentamt
European Patent Office
Office européen des brevets

(11) Veröffentlichungsnummer: **0 391 847**
**A1**

(12) # EUROPÄISCHE PATENTANMELDUNG

(21) Anmeldenummer: **90810246.0**

(22) Anmeldetag: **27.03.90**

(51) Int. Cl.[5]: **C07D 211/88, C07D 405/12, C07D 409/12, C07C 235/74, A01N 43/40**

(30) Priorität: **04.04.89 CH 1237/89**
**26.10.89 CH 3869/89**

(43) Veröffentlichungstag der Anmeldung:
**10.10.90 Patentblatt 90/41**

(84) Benannte Vertragsstaaten:
**AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Moser, Hans, Dr.**
**Hauptstrasse 67b**
**CH-4312 Magden(CH)**
Erfinder: **Pissiotas, Georg, Dr.**
**Am Sonnenrain 71**
**D-7850 Lörrach(DE)**
Erfinder: **Brunner, Hans-Georg, Dr.**
**Wannenstrasse 14**
**CH-4415 Lausen(CH)**

(54) **1-Phenyl-piperidin-2,4-dione mit herbizider Wirkung.**

(57) Die Erfindung betrifft die neuen, herbizid wirksamen 1-Phenyl-piperidin-2,6-dione der Formel

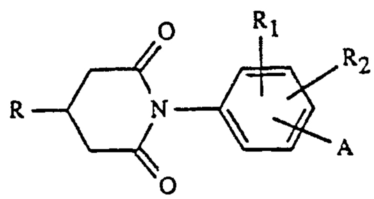 (I),

worin
R Wasserstoff; oder $C_1$-$C_6$-Alkyl; $C_3$-$C_7$-Cycloalkyl; $C_1$-$C_4$-Alkoxy-$C_1$-$C_4$-alkyl; $C_1$-$C_4$-Alkylthio-$C_1$-$C_4$-alkyl; Halogen-$C_1$-$C_7$-alkyl; $C_3$-$C_7$-Alkenyl; $C_3$-$C_7$-Alkinyl;
$R_1$ Wasserstoff; oder Halogen;
$R_2$ Wasserstoff; Cyano; Nitro; oder Halogen;
A Wasserstoff; Cyano; Nitro; oder einen in der Beschreibung näher definierten Aether oder Säurerest bedeutet, die Salze und Komplexe sowie stereoisomeren Formen dieser Verbindungen, sowie Verfahren zur deren Herstellung, agrochemische Mittel, die diese Verbindungen enthalten und ihre Verwendung.

EP 0 391 847 A1

## 1-Phenyl-piperidin-2,4-dione mit herbizider Wirkung

Die Erfindung betrifft die neuen 1-Phenyl-piperidin-2,6-dione der Formel

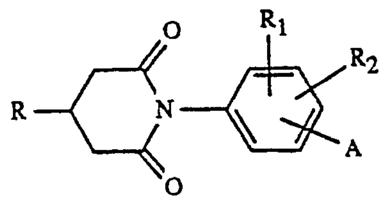

(I),

worin

R Wasserstoff; $C_1$-$C_6$-Alkyl; $C_3$-$C_7$-Cycloalkyl; $C_1$-$C_4$-Alkoxy-$C_1$-$C_4$-alkyl; $C_1$-$C_4$-Alkylthio-$C_1$-$C_4$-alkyl; Halogen-$C_1$-$C_7$-alkyl; $C_3$-$C_7$-Alkenyl; $C_3$-$C_7$-Alkinyl;

$R_1$ Wasserstoff; Halogen;

$R_2$ Wasserstoff; Cyano; Nitro; Halogen;

$R_3$ Halogen; $X'$-$R_5$; Amino; $C_1$-$C_4$-Alkylamino; Di-$C_1$-$C_4$-alkylamino; $C_2$-$C_4$-Halogenalkylamino; Di-$C_2$-$C_4$-halogenalkylamino; $C_1$-$C_4$-Hydroxyalkylamino; Di-$C_1$-$C_4$-hydroxyalkylamino; $C_3$-$C_4$-Alkenylamino; Diallylamino; -N-Pyrrolidino; -N-Piperidino; -N-Morpholino; -N-Thiomorpholino; -N-Piperidazino;

A Wasserstoff; Cyano; Nitro; $COR_3$;

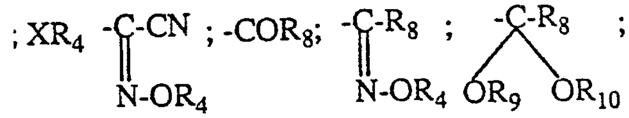

$-\underset{\underset{O}{\|}}{C}-X[CHR_{11}(CH_2)_n]-Si(R_{12})_3$

und $-X-[CHR_{11}(CH_2)_n]-Si(R_{12})_3$;

$R_4$ Wasserstoff, $C_1$-$C_{10}$-Alkyl; $C_1$-$C_4$-Alkoxy-$C_1$-$C_4$-alkyl; $C_1$-$C_4$-Alkylthio-$C_1$-$C_4$-alkyl; Di-$C_1$-$C_4$-alkylamino-$C_1$-$C_4$-alkyl; Halogen-$C_1$-$C_8$-alkyl; $C_2$-$C_8$-Alkenyl; Halogen-$C_2$-$C_8$-alkenyl; $C_3$-$C_8$-Alkinyl; $C_3$-$C_7$-Cycloalkyl; Halogen-$C_3$-$C_7$-cycloalkyl; $C_1$-$C_8$-Alkylcarbonyl; Allylcarbonyl; $C_3$-$C_7$-Cycloalkylcarbonyl; Benzoyl, das unsubstituiert oder am Phenylring gleich oder verschieden bis zu dreifach durch Halogen, $C_1$-$C_4$-Alkyl, Halogen-$C_1$-$C_4$-alkyl, Halogen-$C_1$-$C_4$-alkoxy oder $C_1$-$C_4$-Alkoxy, substituiert ist; Furanoyl; Thiophenoyl; oder $C_1$-$C_4$-Alkyl substituiert durch Phenyl, Halogenphenyl, $C_1$-$C_4$-Alkylphenyl, $C_1$-$C_4$-Alkoxyphenyl, Halogen-$C_1$-$C_4$-alkylphenyl, Halogen-$C_1$-$C_4$-alkoxyphenyl, $C_1$-$C_6$-Alkoxycarbonyl, $C_1$-$C_4$-Alkoxy-$C_1$-$C_8$-alkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl, $C_3$-$C_8$-Alkinylthiocarbonyl, Carbamoyl, Mono-$C_1$-$C_4$-alkylaminocarbonyl, Di-$C_1$-$C_4$-alkylaminocarbonyl, Phenylaminocarbonyl, das unsubstituiert oder am Phenyl gleich oder verschieden bis zu dreifach durch Halogen, $C_1$-$C_4$-Alkyl, Halogen-$C_1$-$C_4$-alkyl, Halogen-$C_1$-$C_4$-alkoxy oder $C_1$-$C_4$-Alkoxy oder einfach durch Cyano oder Nitro substituiert ist; Dioxolan-2-yl, das unsubstituiert ist oder durch ein oder zwei $C_1$-$C_4$-Alkylreste substituiert ist; oder Dioxan-2-yl, das unsubstituiert ist oder durch ein oder zwei $C_1$-$C_4$-Alkylreste substituiert ist;

$C_1$-$C_4$-Alkyl, das durch Cyano, Nitro, Carboxyl, $C_1$-$C_8$-Alkylthio-$C_2$-$C_8$-alkoxycarbonyl, $C_1$-$C_8$-Alkoxy-$C_2$-$C_8$-alkoxycarbonyl substituiert ist;

X Sauerstoff oder Schwefel;

$X'$ Sauerstoff oder Schwefel;

$R_5$ Wasserstoff, $C_1$-$C_{10}$-Alkyl; $C_1$-$C_4$-Alkoxy-$C_1$-$C_4$-alkyl; Halogen-$C_1$-$C_8$-alkyl; $C_1$-$C_{10}$-Alkylthio-$C_1$-$C_4$-alkyl; Di-$C_1$-$C_4$-alkylamino-$C_1$-$C_4$-alkyl; Cyano-$C_1$-$C_8$-alkyl; $C_3$-$C_8$-Alkenyl; Halogen-$C_3$-$C_8$-alkenyl; $C_3$-$C_8$-Alkinyl; $C_3$-$C_7$-Cycloalkyl; $C_3$-$C_7$-Cycloalkyl-$C_1$-$C_4$-alkyl; Halogen-$C_3$-$C_7$-cycloalkyl, Benzyl, das unsubstituiert oder am Phenylring gleich oder verschieden bis zu dreifach durch Halogen, $C_1$-$C_4$-Alkyl; Halogen-$C_1$-$C_4$-alkyl, Halogen-$C_1$-$C_4$-alkoxy oder $C_1$-$C_4$-alkoxy substituiert ist; Alkali-, Erdalkali-Ionen und Ammoniumionen; oder die Gruppe $-[CHR_6(CH_2)_n]-COOR_7$;

$R_6$ Wasserstoff; $C_1$-$C_4$-Alkyl;

$R_7$ Wasserstoff; $C_1$-$C_6$-Alkyl; $C_3$-$C_8$-Alkenyl; $C_3$-$C_8$-Alkinyl; $C_1$-$C_8$-Alkoxy-$C_2$-$C_8$-alkyl; $C_1$-$C_8$-Alkylthio-$C_2$-$C_8$-alkyl; $C_3$-$C_7$-Cycloalkyl;

$R_8$ Wasserstoff; $C_1$-$C_4$-Alkyl; Halogen-$C_1$-$C_4$-alkyl; $C_1$-$C_4$-Alkoxy-$C_1$-$C_4$-alkyl;

$R_9$ und $R_{10}$ unabhängig voneinander jeweils $C_1$-$C_4$-Alkyl; $C_2$-$C_4$-Halogenalkyl oder $C_2$-$C_8$-Alkoxyalkyl, oder

$R_9$ und $R_{10}$ zusammen eine Aethylen- oder Propylenbrücke oder einen 1,2-Cyclohexanylenkörper, wobei diese Gruppen unsubstituiert sind oder durch ein bis zwei Reste aus der Gruppe $C_1$-$C_4$-Alkyl, $C_1$-$C_4$-Halogenalkyl oder $C_1$-$C_4$-Hydroxyalkyl substituiert sind;

$R_{11}$ Wasserstoff, $C_1$-$C_5$-Alkyl, $C_3$-$C_7$-Alkenyl;

$R_{12}$ unabhängig voneinander Wasserstoff, $C_1$-$C_8$-Alkyl und

n 0,1,2,3 oder 4

bedeutet unter Einschluss der Salze oder Komplexe der Verbindungen der Formel I mit Säuren, Basen oder Komplexbildnern, sowie der stereoisomeren Verbindungen der Verbindungen der Formel I.

In den in dieser Beschreibung verwendeten Definitionen umfassen die angegebenen generischen Begriffe, sowie die durch Kombination einzelner Unterbegriffe erhältlichen Einzelbedeutungen der Substituenten, beispielsweise die folgenden Einzelsubstituenten, wobei diese Aufzählung keine Einschränkung der Erfindung darstellt.

Alkyl ist Methyl, Ethyl, n-Propyl, iso-Propyl, n-Butyl, sec.-Butyl, iso-Butyl, tert.-Butyl sowie die isomeren Pentyle und Hexyle. Bevorzugt sind die $C_1$-$C_4$-Alkylradikale.

Halogen: Fluor, Chlor, Brom und Jod, vorzugsweise Fluor, Chlor und Brom.

Alkoxy: Methoxy, Aethoxy, Propyloxy, i-Propyloxy, n-Butyloxy, i-Butyloxy, s-Butyloxy und t-Butyloxy, vorzugsweise Methoxy oder Aethoxy.

Halogenalkyl: Fluormethyl, Difluormethyl, Trifluormethyl, Chlormethyl, Dichlormethyl, Trichlormethyl, 2,2,2-Trifluoräthyl, 2-Fluoräthyl, 2-Chloräthyl und 2,2,2-Trichloräthyl, vorzugsweise Chlormethyl, 2-Chloräthyl und Trifluormethyl.

Halogenalkoxy: Fluormethoxy, Difluormethoxy, Trifluormethoxy, 2,2,2-Trifluoräthoxy, 1,1,2,2-Tetrafluoräthoxy, 2-Fluoräthoxy, 2-Chloräthoxy und 2,2,2-Trichloräthoxy, vorzugsweise Difluormethoxy, 2-Chloräthoxy und Trifluormethoxy.

Alkoxycarbonyl: Methoxycarbonyl, Aethoxycarbonyl, n-Propyloxycarbonyl, i-Propyloxycarbonyl und n-Butyloxycarbonyl, vorzugsweise Methoxycarbonyl und Aethoxycarbonyl.

Alkoxyalkyl: Methoxymethyl, Aethoxymethyl, Propyloxymethyl, Methoxyäthyl, Aethoxyäthyl, Propyloxyäthyl, Methoxypropyl, Aethoxypropyl oder Propyloxypropyl.

Alkylthioalkyl: Methylthiomethyl,Aethylthiomethyl, Methylthioäthyl, Aethylthioäthyl, oder Isopropylthioäthyl.

Alkylaminoalkyl: Methylaminoäthyl, Dimethylaminoäthyl, Aethylaminoäthyl oder Diäthylaminoäthyl.

Cyanoalkyl: Cyanomethyl, Cyanoäthyl oder Cyanopropyl.

Alkenyl: Allyl, 2-Butenyl, 3-Butenyl oder Methallyl, vorzugsweise aber Allyl.

Alkinyl: Propargyl, 2-Butinyl oder 3-Butinyl, vorzugsweise aber Propargyl.

Cycloalkyl: Cyclopropyl, Cyclobutyl, Cyclopentyl, Cyclohexyl oder Cycloheptyl. Bevorzugt sind Cyclopentyl oder Cyclohexyl.

Halogencycloalkyl: 2,2-Dichlorcyclopropyl oder Pentachlorcyclohexyl.

Alkylsulfonyl: Methylsulfonyl, Aethylsulfonyl, Propylsulfonyl oder Butylsulfonyl. Bevorzugt sind Methyl- und Aethylsulfonyl.

Cycloalkoxycarbonyl: Cyclopentyloxycarbonyl oder Cyclohexyloxycarbonyl.

Phenyl, auch als Teil eines Substituenten wie Phenoxy, Phenylthio, Phenoxycarbonyl, Phenylaminocarbonyl, Benzyl oder Benzoyl, kann im allgemeinen unsubstituiert oder durch weitere Substituenten substituiert vorliegen. Die Substituenten können dann in ortho-, meta- und/oder para-Stellung stehen. Bevorzugte Substituentenstellungen sind die ortho- und para-Position zur Ringverknüpfungsstelle. Bevorzugte Substituenten sind Halogenatome.

In den weiteren Substituenten, welche aus mehreren Grundelementen zusammengesetzt sind, haben die Teilelemente die oben an Beispielen erläuterten Bedeutungen. Diese Aufzählungen bedeuten auch in diesen Fällen keine Einschränkdung der Erfindung: sie haben illustrierenden Charakter.

Von den erfindungsgemässen Verbindungen sind diejenigen bevorzugt, welche einer der folgenden Formeln entsprechen:

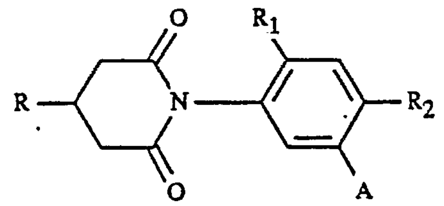

(Ia),

worin
R Wasserstoff; oder $C_1$-$C_6$-Alkyl; Halogen-$C_1$-$C_7$-alkyl;
$R_1$ Wasserstoff; oder Halogen;
$R_2$ Wasserstoff; Cyano; Nitro; oder Halogen;
A Wasserstoff; Cyano; Nitro; $COR_3$; oder X-$R_4$;
$R_3$ Halogen; oder $X'$-$R_5$;
$R_4$ Wasserstoff; $C_1$-$C_6$-Alkyl; $C_3$-$C_6$-Alkenyl; oder $C_3$-$C_6$-Alkinyl;
X Sauerstoff; oder Schwefel;
$X'$ Sauerstoff; oder Schwefel;
$R_5$ Wasserstoff; $C_1$-$C_6$-Alkyl; oder die Gruppe $-[CHR_6(CH_2)_n]-COOR_7$;
$R_6$ Wasserstoff; oder $C_1$-$C_4$-Alkyl;
$R_7$ Wasserstoff; oder $C_1$-$C_6$-Alkyl; und
n die Zahl 0, 1, 2, 3- oder 4;
bedeutet unter Einschluss der Salze oder Komplexe der Verbindungen dieser Formel mit Säuren, Basen oder Komplexbildnern;

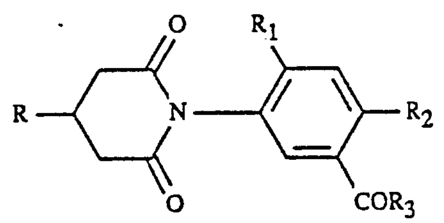

(Ib),

worin R, $R_1$, $R_2$ und $R_3$ die unter der Formel I gegebene Bedeutung haben;

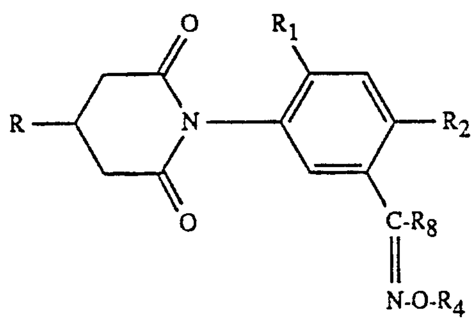

(Ic),

worin R, $R_1$, $R_2$, $R_8$ und $R_4$ die unter Formel I gegebene Bedeutung haben;

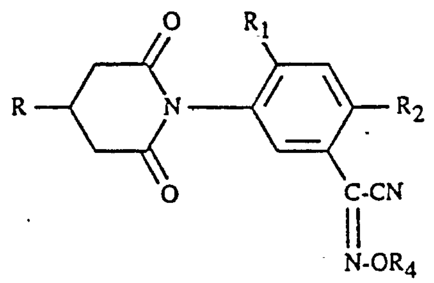

(Id),

worin R, $R_1$, $R_2$ und $R_4$ die unter der Formel I gegebene Bedeutung haben;

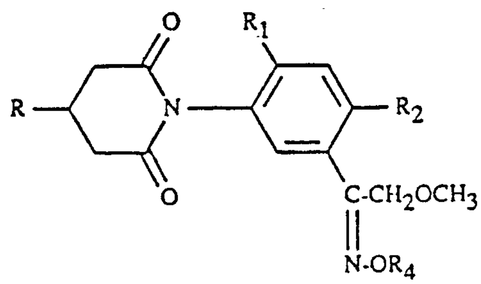

(Ie),

worin R, $R_1$, $R_2$ und $R_4$ die unter Formel I gegebene Bedeutung haben;

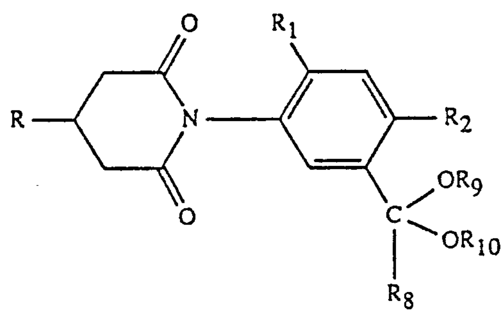

(If),

worin R, $R_1$, $R_2$, $R_8$, $R_9$ und $R_{10}$ die unter der Formel I gegebene Bedeutung haben;

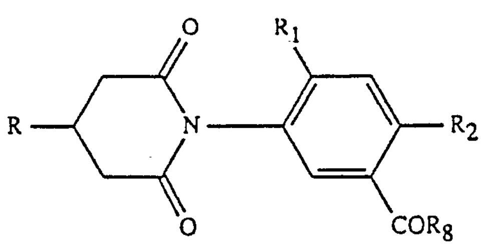

(Ig),

worin R, $R_1$, $R_2$, und $R_8$ die unter der Formel I gegebene Bedeutung haben;

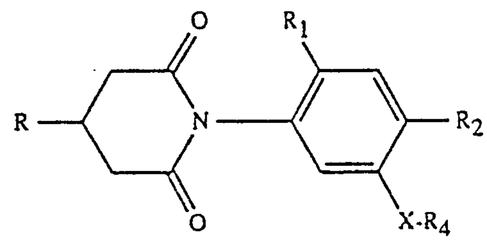

(Ih),

worin R, $R_1$, $R_2$, $R_4$ und X die unter Formel I gegebene Bedeutung haben;

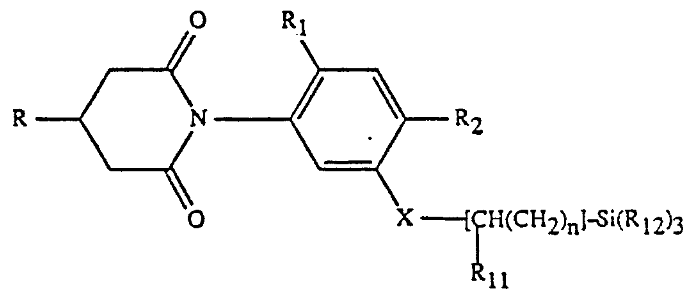

(Ii),

worin n, R, $R_1$, $R_2$, $R_{11}$ und $R_{12}$ die unter der Formel I gegebene Bedeutung haben;

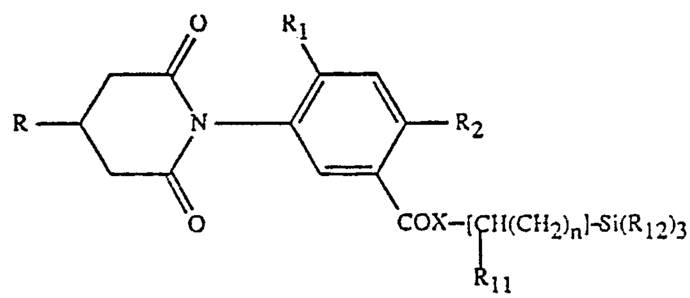

(Ij),

worin n, $R_1$, $R_2$, $R_{11}$, $R_{12}$ und X die unter der Formel I gegebene Bedeutung haben;

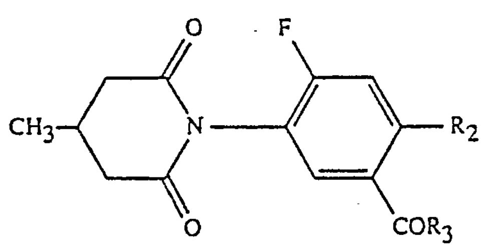

(Il)

worin R, $R_1$, $R_2$ und $R_3$ die unter Formel I gegebene Bedeutung haben;

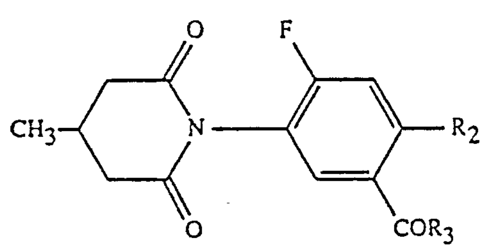

(Il)

worin $R_2$ Chlor oder Brom bedeutet und $R_3$ die unter der Formel I gegebene Bedeutung hat;

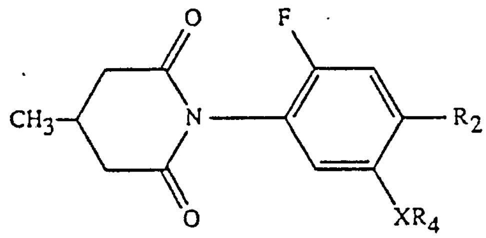

(Im)

worin $R_2$ Chlor oder Brom bedeutet und X und $R_4$ die unter der Formel I gegebene Bedeutung haben;

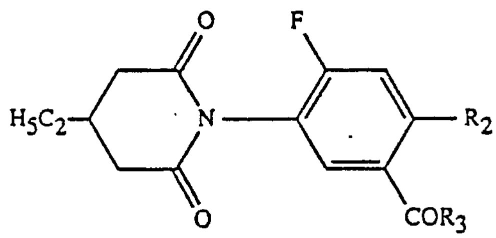

(In)

worin $R_2$ Chlor oder Brom bedeutet und $R_3$ die unter der Formel I gegebene Bedeutung hat;

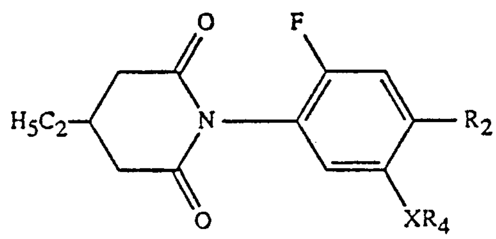

(Io)

worin $R_2$ Chlor oder Brom bedeutet und X und $R_4$ die unter der Formel I gegebene Bedeutung haben;

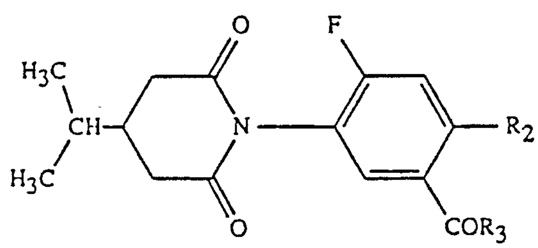

(Ip)

worin $R_2$ Chlor oder Brom bedeutet und $R_3$ die unter der Formel I gegebene Bedeutung hat;

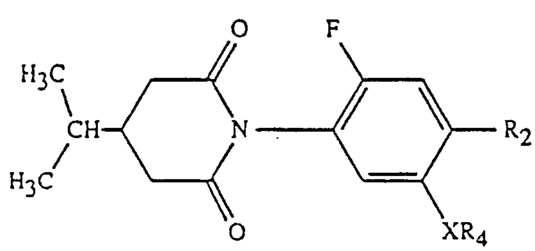

(Iq)

worin $R_2$ Chlor oder Brom bedeutet und X und $R_4$ die unter der Formel I gegebene Bedeutung hat;

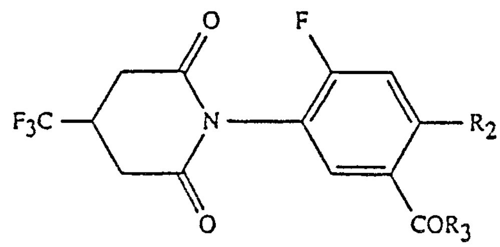

(Ir)

worin $R_2$ Chlor oder Brom bedeutet und $R_3$ die unter der Formel I gegebene Bedeutung hat;

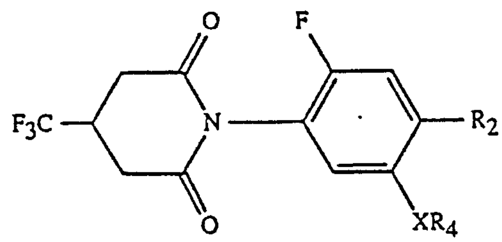

(Is)

worin $R_2$ Chlor oder Brom bedeutet und X und $R_4$ die unter Formel I gegebene Bedeutung haben.

Die Erfindung betrifft neben den Salzen und Komplexen, die diese Verbindungen mit Säuren, Basen und Komplexbildnern eingehen, auch alle möglichen Stereoisomeren, die in Form von Enantiomeren, Diastereomeren oder deren Gemische vorliegen.

Die Verbindungen der Formel I sind neu. Sie können z.B. hergestellt werden durch Umsetzung von Glutarsäureanhydriden der Formel II mit Aminen der Formel III, worin die Reste R, $R_1$, $R_2$ und A wie unter Formel I definiert sind, zu einem

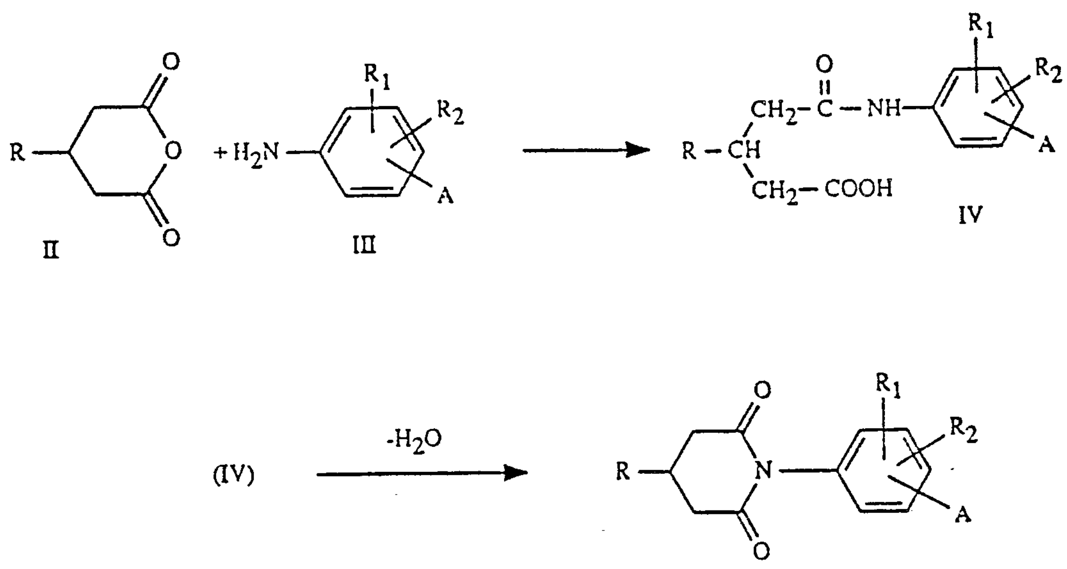


II III IV (IV) I

Glutarsäuremonoanilid der Formel IV und anschliessender Cyclisierung des Monoamids IV mit einem Kondensationsmittel zu einem Piperidin-2,6-dion der Formel I.

Die Verbindungen der Formel II und III sind bekannt oder können analog zu bekannten Verfahren dargestellt werden. Die Glutarsäuremonoamide der Formel IV dagegen sind neu. Sie sind wertvolle Zwischenverbindungen zur Herstellung der erfindungsgemässen Endprodukte der Formel I. Die Erfindung betrifft somit auch die neuen Glutaramide der Formel IV, worin die Reste R, $R_1$, $R_2$ und A wie unter Formel

I definiert sind.

Zur Synthese der in 3-Position substituierten Glutarsäureanhydriden sind in der Literatur verschiedene Wege beschrieben.

Als geeignet für die Herstellung der Verbindungen der Formel II erweist sich unter anderem die in Schema 1 dargestellte Reaktionssequenz, die von einfachsten, allgemein verfügbaren Synthesebausteinen (Aldehyd und Cyanessigsäureester) ausgeht:

Schema 1

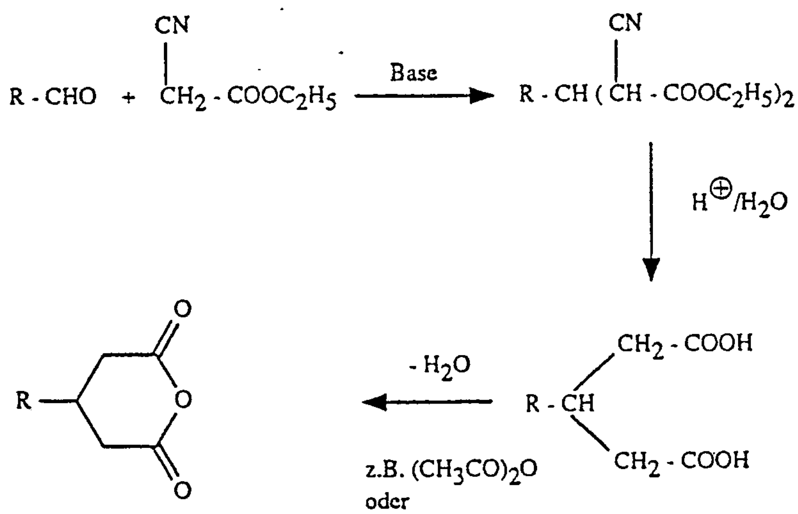


II

Das Verfahren gemäss Schema 1 ist unter anderem in folgenden Literaturstellen näher beschrieben: J. Chem. Soc. 117 (1920) 1465; J. Chem. Soc. 123 (1923), 3131; J. Chem. Soc. 1952, 4785; Rec. Trav. Chim. Pays-Bas 84 (1965), 1183 und J. Ind. Chem. Soc. 13 (1936), 322.

Eine andere Verfahrensvariante zur Herstellung der Glutarsäureanhydride II basiert auf einer Malonestersynthese und ist im Schema 2 zusammengefasst:

Schema 2

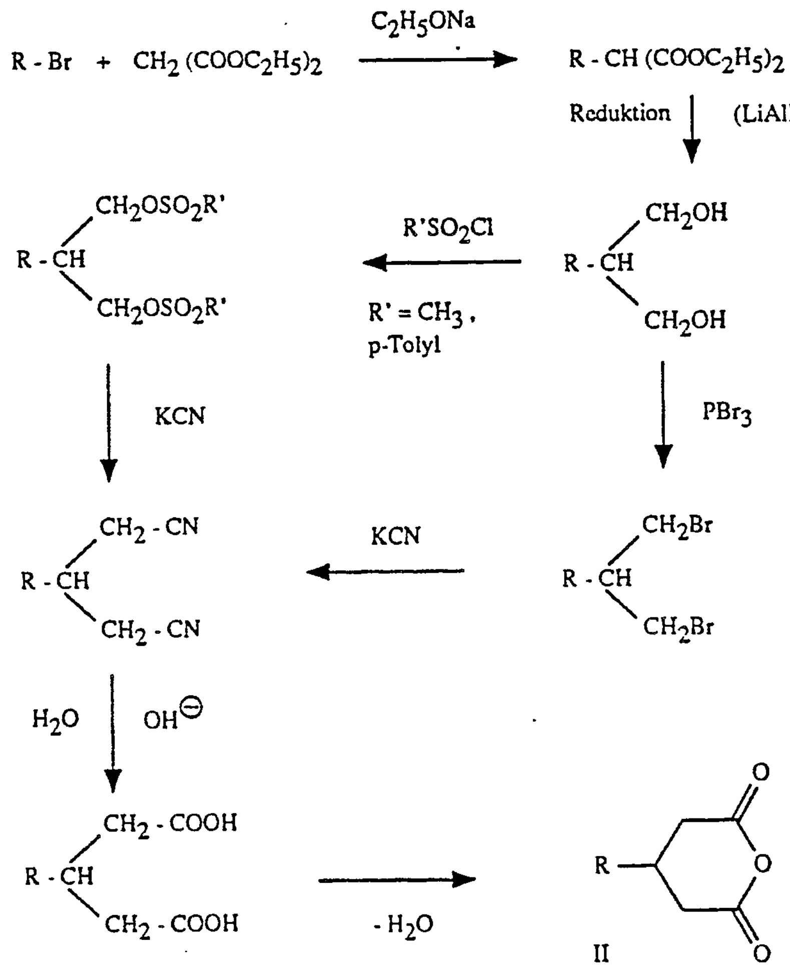


II

Das in Schema 2 skizzierte Verfahren ist unter anderem auf folgenden Literaturstellen bekannt: Chem. Soc. Perkin Trans. I, 1978, 1636; J. Am. Chem. Soc. 95 (1973), 7437; Org. Prep. Proc. Int. 7, (1975), 283.

Die Aniline der Formel III sind aus zahlreichen Patentpublikationen bekannt geworden. Zu nennen sind unter anderem die EP-A 83 055, EP-A 61 741, EP-A 303 573, EP-A 216 243, EP-A 207 894 oder die EP-A 190 755.

Darüber hinaus sind auch einige der unter die Formel I fallenden Verbindungen in besonderer Weise geeignete Ausgangsverbindungen zur Herstellung anderer Derivate der Formel I.

Die Erfindung betrifft somit auch Verfahren zur Herstellung von Piperidin-2,6-dionen der Formel Ik

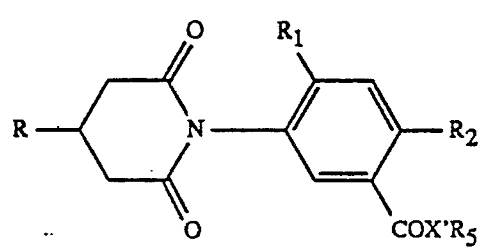

(Ik),

worin die Reste R, $R_1$, $R_2$, $R_5$ und X′ wie zuvor definiert sind,dadurch gekennzeichnet, dass man ein Carbonsäurehalogenid der Formel VIII, worin die Reste R, $R_1$ und $R_2$ wie zuvor definiert sind und Hal für Chlor, Brom oder Jod steht, mit einem Alkohol oder Merkaptan der Formel V, umsetzt, wobei diese

Reaktion vorzugsweise in

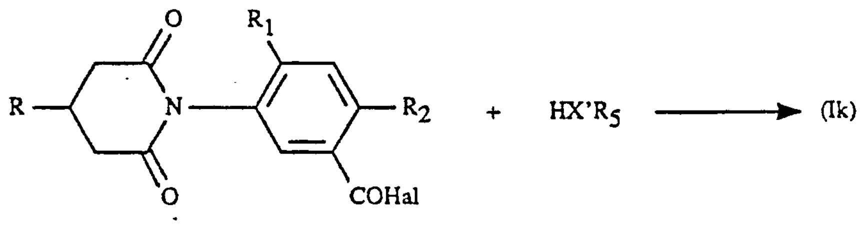


(VIII) (V)

Gegenwart einer äquimolaren Menge einer Base durchgeführt wird.

Die Carbonsäurederivate der Formel Ik

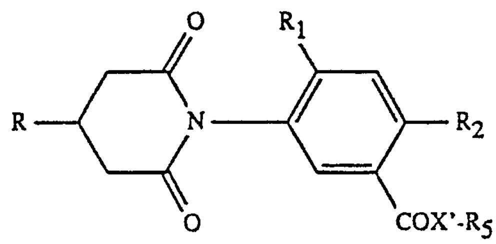

(Ik),

worin die Reste R, $R_1$, $R_2$ $X'$ und $R_5$ wie zuvor definiert sind bedeutet, können ausgehend von den Carbonsäuren der Formel VII durch direkte Veresterung

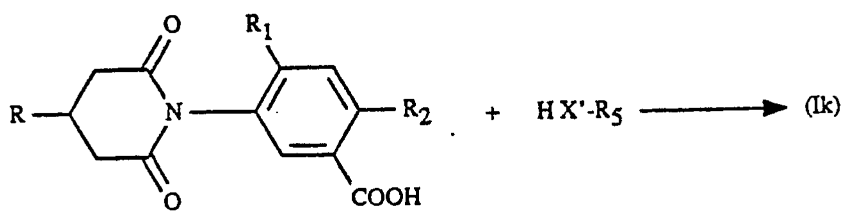


(VII) (V)

mit den Alkoholen oder Mercaptanen der Formel V in Gegenwart einer Säure oder eines wasserbindenden Mittels hergestellt werden.

Als wasserbindendes Mittel in vorstehender Reaktion hat sich u.a. Dicyclohexylcarbodiimid bewährt.

Schliesslich sind die Ether der Formel Ih

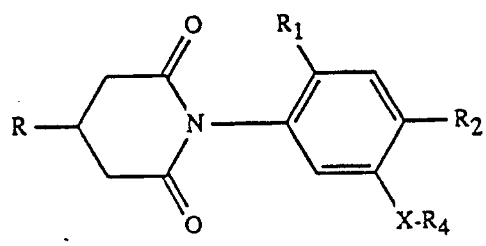

(Ih),

worin die Reste R, $R_1$, $R_2$, $R_4$ und X wie zuvor definiert sind, herstellbar durch die Umsetzung von Phenolen oder Thiophenolen der Formel IX mit Verbindungen der Formel X,

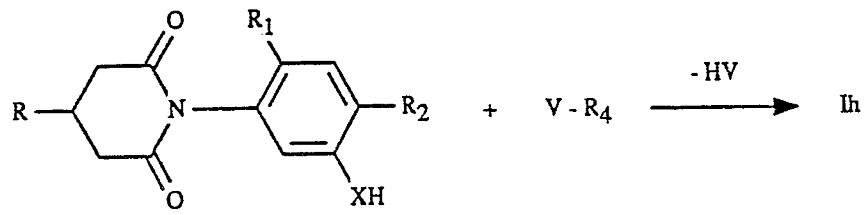


(IX) (X)

in der V für eine unter den Reaktionsbedingungen substituierbare nucleofuge Gruppe, wie Halogen, $C_1$-$C_4$-Alkylsulfonyl oder Arylsulfonyl steht, unter Einwirkung einer Base.

Die Verbindungen der Formel I werden erfindungsgemäss hergestellt, indem man ein Glutarsäureanhydrid der Formel II

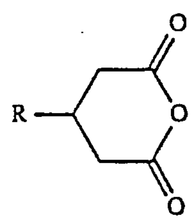

(II),

worin R die unter Formel I gegebene Bedeutung hat, mit einem Anilin der Formel III

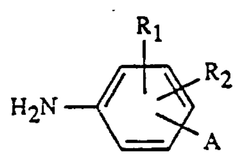

(III),

worin A, $R_1$ und $R_2$ die unter Formel I gegebene Bedeutung hat, zum Glutarsäuremonoanilid der Formel IV kondensiert

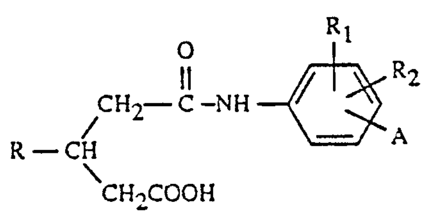

(IV),

worin A, R, $R_1$ und $R_2$ die unter Formel I gegebene Bedeutung haben und anschliessend mit einem Kondensationsmittel zum 1-Phenylpiperidin-2,6-dione der Formel I cyclisiert,

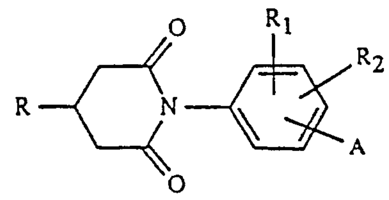

(I).

Als Kondensationsmittel für diese Cyclokondensation kommen unter anderem Säureanhydride, wie Essigsäureanhydrid, Säurehalogenide, wie Acetylchlorid oder Thionylchlorid in Frage.

Mit Vorteil führt man die obige Kondensationsreaktion in einem inerten organischen Lösungsmittel aus. Die Reaktionstemperatur liegt im allgemeinen zwischen der Raumtemperatur und der Siede- temperatur des Reaktionsgemisches, vorzugsweise wird die Reaktionsmischung zum Rückfluss erhitzt. Die Kondensationsreaktion kann durch Zusatz von Kondensationskatalysatoren und Entfernung des entstehenden Reaktionsproduktes Wasser beschleunigt werden. Eine gleiche Wirkung erzielt man durch Zusatz von wasserentziehenden Mitteln, wie beispielsweise Schwefelsäure.

Als Lösungsmittel eignen sich insbesondere höhersiedende Kohlenwasserstoffe, Ester und Amide, höhersiedende Ketone und Aether. Beispiele dafür sind Benzol, Toluol, Xylol, Dimethylformamid, Dimethylacetamid, Ethylacetat, Diisopropyläther, Aethylenglykoldimethyläther, Tetrahydrofuran, Dioxan oder 2-Butanon, Chloroform, Tetrachlorkohlenstoff, oder Dichlormethan oder Methylenchlorid.

Obwohl die oben skizzierte Synthese der Verbindungen der Formel I aus einem Anhydrid der Formel II und einem Anilin der Formel III in allen Fällen durchführbar ist, kann es aus ökonomischen oder verfahrenstechnischen Gründen sinnvoll sein, bestimmte Verbindungen der Formel I in andere Derivate der Formel I zu überführen. Dabei kommen für den Fachmann bekannte Verfahren, wie beispielsweise Oxidation, Reduktion, Veresterung, Verseifung oder Amidierung in Frage. Einige Beispiele für derartige Umwandlungen von bestimmten Wirkstoffen der Formel I in andere Wirkstoffe der Formel I sind in den folgenden Schemata 3 bis 5 aufgeführt.

Schema 3:

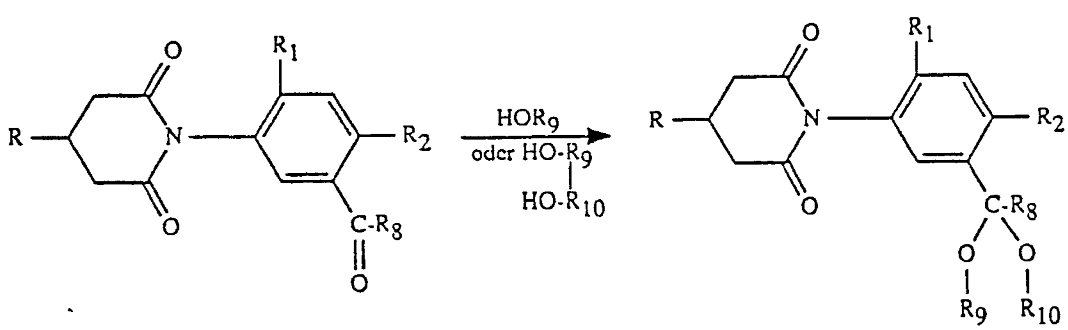

Schema 4:

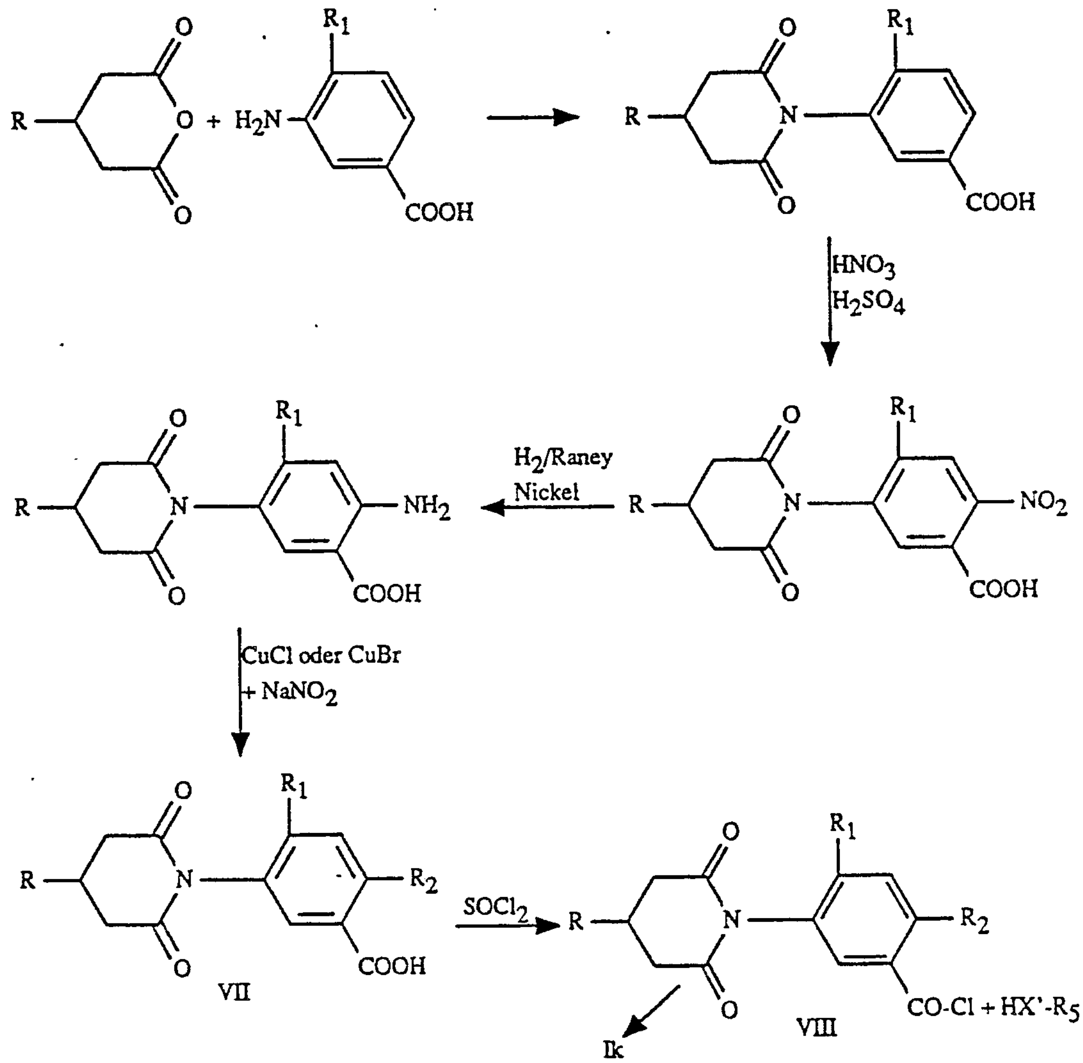


VII

VIII

Ik

Die Säurechloride der Formel VIII, in denen $R_1$ Wasserstoff oder Fluor, $R_2$ Chlor oder Brom, und R wie in Formel I definiert bedeuten, sind wichtige Zwischenprodukte bei der Herstellung von Imiden der Formel I. Sie und ihre Herstellung sind ebenfalls ein Gegenstand dieser Erfindung.

Schema 5: Hal: Chlor, Brom

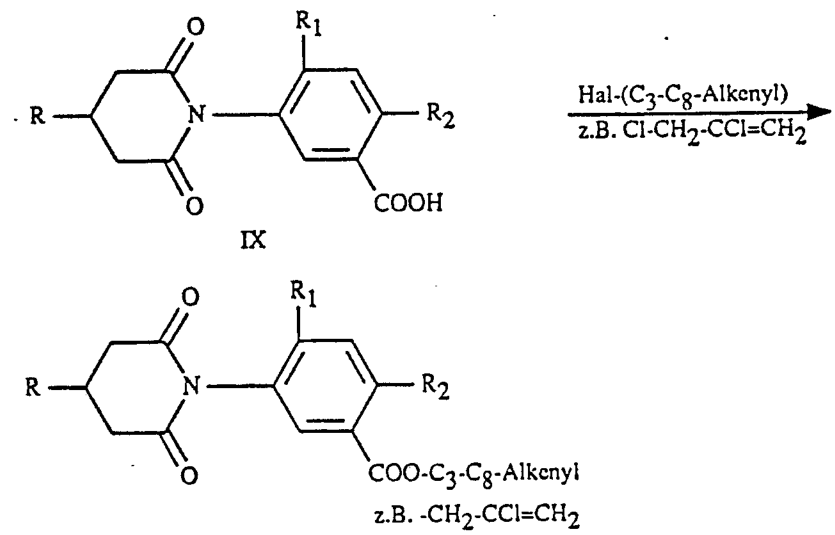


IX

Die Erfindun betrifft auch alle möglichen Stereoisomeren, die in Form von Enantiomeren, Diastereomeren oder deren Gemische vorliegen.

Die Verbindungen der Formel Ib, Id und If sind somit auch wertvolle Zwischenverbindungen zur Herstellung höherer funktionalisierter Verbindungen der Formel I.

Die vorstehend aufgeführten Verfahren können analog zu literaturbekannten Verfahren durchgeführt werden. Die in diesen Verfahren bevorzugten Reaktionsbedingungen, wie Temperatur, molare Verhältnisse der Edukte, Reaktionsführung. Lösungsmittel, gegebenenfalls erforderliche Reagentien, wie Säuren, Basen, wasserbindende Mittel usw., sind dem Fachmann geläufig.

Die Verbindungen der Formel I sind stabile Verbindungen bei deren Handhabung keine besonderen Vorsichtsmassnahmen notwendig sind.

Die Verbindungen der Formel I sind hochaktive Herbizide, die sich bei entsprechenden Aufwandmengen in vorteilhafter Weise auch als Selektivherbizide zur Unkrautbekämpfung in Nutzpflanzenkulturen eignen, Kulturpflanzen wie Getreide (wie Roggen, Gerste, Hafer, Weizen), Mais, Hirse, Reis, Baumwolle, Soja und Sonnenblumen bleiben bei niedrigen Aufwandmengen praktisch ungeschädigt. Bei gesteigerten Aufwandmengen werden die Kulturpflanzen nur geringfügig in ihrem Wachstum beeinflusst. Werden sehr hohe Aufwandmengen appliziert, entfalten die Substanzen der Formel I totalherbizide Eigenschaften.

Sie selektiv-herbizide Wirkung der erfindungsgemässen Verbindungen wird sowohl bei der preemergenten als auch der postemergenten Anwendung festgestellt. Diese Wirkstoffe können daher im Vorauflaufverfahren und im Nachauflaufverfahren zur selektiven Unkrautbekämpfung mit gutem Erfolg verwendet werden. Bevorzugt werden die Verbindungen der Formel I aber im Vorauflauf eingesetzt.

Die Erfindung betrifft auch herbizide Mittel, welche einen neuen Wirkstoff der Formel I enthalten, sowie Verfahren zur pre- und postemergenten Unkrautbekämpfung.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

So können die Aktivsubstanzen der Formel I auch auf mineralische Dünger aufgebracht (aufgebeizt) werden.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen

$C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder ethylether, Ketone wie Cyclo hexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kationen- und/oder anionenaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylen-sorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:
"1987 International Mc Cutcheon's Emulsifiers and Detergents",
Glen Rock, N.J., USA.

Dr. Helmut Stache "Tensid Taschenbuch"
Carl Hanser Verlag, München/Wien 1981.

Die Zubereitungen enthalten in der Regel 0,1 bis 95 %, insbesondere 0,1 bis 80 %, Wirkstoff der Formel I, 1 ist 99,9 % eines festen oder flüssigen Zusatzstoffs und 0 bis 25 %, insbesondere 0,1 bis 25 % eines Tensides.

Insbesondere setzen sich bevorzugte Formulierungen folgendermassen zusammen:
(% = Gewichtsprozent)

Emulgierbare Konzentrate:
Wirkstoff der Formel I: 1 bis 20 %, bevorzugt 5 bis 10 %
oberflächenaktives Mittel: 5 bis 30 %, vorzugsweise 10 bis 20 %
flüssiges Trägermittel: 50 bis 94 %, vorzugsweise 70 bis 85 %.

Stäube:
Wirkstoff der Formel I: 0,1 bis 10 %, vorzugsweise 0,1 bis 1 %
festes Trägermittel: 99,9 bis 90 %, vorzugsweise 99,9 bis 99 %.

Suspensions-Konzentrate:
Wirkstoff der Formel I: 5 bis 75 %, vorzugsweise 10 bis 50 %
Wasser: 94 bis 25 %, vorzugsweise 90 bis 30 %
oberflächenaktives Mittel: 1 bis 40 %, vorzugsweise 2 bis 30 %.

Benetzbare Pulver:
Wirkstoff der Formel I: 0,5 bis 90 %, vorzugsweise 1 bis 80 %
oberflächenaktives Mittel: 0,5 bis 20 %, vorzugsweise 1 bis 15 %
festes Trägermittel: 5 bis 95 %, vorzugsweise 15 bis 90 %.

Granulate:
Wirkstoff der Formel I: 0,5 bis 30 %, vorzugsweise 3 bis 15 %
festes Trägermittel: 99,5 bis 70 %, vorzugsweise 97 bis 85 %.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### H.1. 1-(4-Chlor-2-fluor-5-hydroxyphenyl)-4-methyl-piperidin-2,6-dion

### H.1.1. N-(4-Chlor2-fluor-5-hydroxyphenyl)-3-methylglutarsäuremonoamid

16,15 g (0,1 Mol) 4-Chlor-2-fluor-5-hydroxy-anilin und 12,8 g (0,1 Mol) 3-Methylglutarsäureanhydrid werden in 180 ml Benzol suspendiert und unter Rühren zum Rückfluss erhitzt. Für kurze Zeit bildet sich eine klare Lösung, aus der beige-braune Kristalle ausfallen. Nach 16 Stunden wird das Reaktionsgemisch auf Raumtemperatur abgekühlt und von dem Niederschlag abfiltriert. Das so erhältliche Produkt wird aus Essigsäureethylester und Aktivkohle umkristallisiert.

Man isoliert 25,9 g der Titelverbindung der Formel

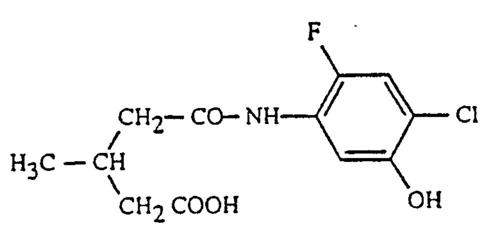

als Kristalle vom Smp. 155-157° C.

H.1.2. 1-(4-Chlor-2-fluor-5-hydroxyphenyl)-4-methyl-piperidin-2,6-dion

24,0 g (0,083 Mol) des nach Beispiel H.1.1 erhältlichen Glutarsäuremonoamids werden in 200 ml Dichlorethan suspensiert. Unter Rühren werden 1 ml Dimethylformamid und 12,2 ml (0,166 Mol) Thionylchlorid zugegeben und das Gemisch bei 60° Badtemperatur gerührt, bis die Chlorwasserstoffgasentwicklung beendet ist. Danach wird das Reaktionsgemisch eingedampft und das zurückbleibende Oel mit einer Mischung aus Diethylether und Eiswasser aufgenommen. Die Etherphase wird nacheinander mit Eiswasser, 10 %-iger Natriumbicarbonätlösung, Eiswasser und gesättigter Kochsalzlösung gewaschen und danach über Natriumsulfat getrocknet. Der nach dem Abdampfen des Ethers zurückbleibende dunkle Festkörper wird bei 0° C mit Diethylether verrührt, abgenutscht und aus Essigester/Petrolether umkristallisiert.

Man isoliert 16,8 g der Titelverbindung der Formel

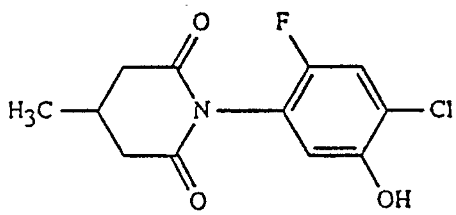

als Kristalle vom Smp. 174-178° C (Verb. No, 1.11).

H.2. 1-(4-Chlor-2-fluor-5-propargyloxyphenyl)-4-methyl-piperidin-2,6-dion

8,1 g des nach Beispiel H.1.2 erhältlichen N-(4-Chlor-2-fluor-5-hydroxyphenyl)-4-methyl-piperidin-2,6-dions werden mit 4,6 g (0,033 Mol) Kaliumcarbonat in 100 ml Methylethylketon für 2 Stunden auf 50° C erwärmt und gut durchgerührt. Zu der so erhältlichen Suspension werden bei Raumtemperatur 2,5 ml (0,033 Mol) Propargylbromid getropft. Das Reaktionsgemisch wird dann erneut auf 50° C erwärmt und für weitere 12 Stunden bei dieser Temperatur belassen. Nach dem Erkalten wird von dem Ungelösten abfiltriert und das Filtrat am Rotationsverdampfer eingeengt. Der Rückstand wird in Essigester aufgenommen und mit Wasser und gesättigter Kochsalzlösung extrahiert und die organische Phase über Natriumsulfat getrocknet und am Vakuum eingedampft. Der Rückstand wird aus Methanol umkristallisiert.

Man isoliert 6,8 g der Titelverbindung der Formel

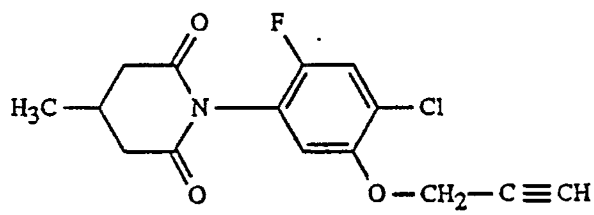

als Kristalle vom Smp. 136-138° C (Verb. No. 1.02).

Analog zu den vorstehenden Herstellungsbeispielen werden die Verbindungen der Tabellen 1-11 hergestellt:

Verbindungen der Formel

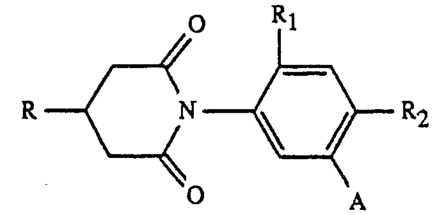

(Ia)

Tabelle 1

| | R | $R_1$ | $R_2$ | A | |
|---|---|---|---|---|---|
| 1.01 | H | F | Cl | H | |
| 1.02 | H | Cl | Cl | H | |
| 1.03 | H | F | Br | H | |
| 1.04 | H | F | Br | CN | |
| 1.05 | H | F | Cl | CN | |
| 1.06 | H | F | Cl | $NO_2$ | |
| 1.07 | H | Cl | Cl | H | |
| 1.08 | H | F | CN | H | |
| 1.09 | $CH_3$ | F | Cl | H | $n_D^{25}$ 1.5336 |
| 1.10 | $CH_3$ | F | Br | CN | |
| 1.11 | $CH_3$ | F | Cl | CN | |
| 1.12 | $CH_3$ | F | Cl | $NO_2$ | |
| 1.13 | $CH_3$ | F | Br | H | |
| 1.14 | $C_2H_5$ | F | Cl | H | |
| 1.15 | $C_2H_5$ | F | Br | CN | |
| 1.16 | $C_2H_5$ | F | Cl | CN | |
| 1.17 | $C_2H_5$ | F | Cl | $NO_2$ | |
| 1.18 | $C_2H_5$ | Cl | Cl | H | |
| 1.19 | $C_3H_7$(i) | F | Cl | H | |
| 1.20 | $C_3H_7$(i) | F | Br | CN | |
| 1.21 | $C_3H_7$(i) | F | Cl | CN | |
| 1.22 | $CF_3$ | F | Cl | H | |
| 1.23 | $CF_3$ | F | Cl | $NO_2$ | |
| 1.24 | $CF_3$ | F | Cl | CN | |
| 1.25 | $C_3H_7$(i) | F | Cl | $NO_2$ | |
| 1.26 | 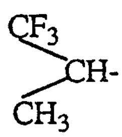 | F | Cl | $NO_2$ | |
| 1.27 | 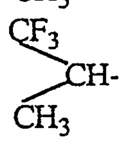 | F | Br | $NO_2$ | |

Verbindungen der Formel

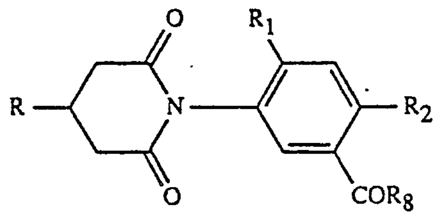

(Ib)

Tabelle 2

| | R | $R_1$ | $R_2$ | $R_8$ | |
|---|---|---|---|---|---|
| 2.01 | H | F | Cl | H | |
| 2.02 | H | F | Cl | $CH_3$ | |
| 2.03 | $CH_3$ | F | Cl | H | |
| 2.04 | $CH_3$ | F | Cl | $CH_3$ | |
| 2.05 | $CH_3$ | F | Cl | $CH_2$-Cl | |
| 2.06 | $C_2H_5$ | F | Cl | H | |
| 2.07 | $C_2H_5$ | F | Cl | $CH_3$ | |
| 2.08 | $C_2H_5$ | F | Cl | $CH_2$-Cl | |
| 2.09 | $C_3H_7$(i) | F | Cl | H | |
| 2.10 | $C_3H_7$(i) | F | Cl | $CH_3$ | |
| 2.11 | 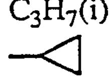 | F | Cl | H | |
| 2.12 | $CH_2$-$OCH_3$ | F | Cl | H | |
| 2.13 | -$CH_2$-CH=$CH_2$ | F | Cl | H | |
| 2.14 | $CF_3$ | F | Cl | $CH_3$ | |
| 2.15 | 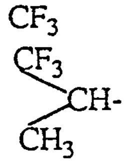 | F | Cl | $CH_3$ | |

Verbindungen der Formel

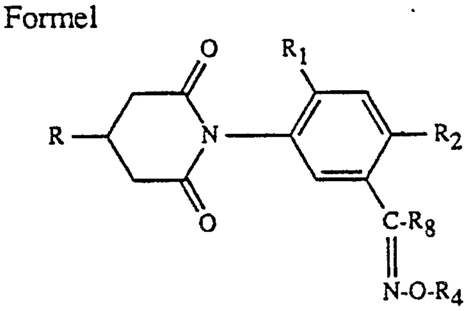

(Ic),

Tabelle 3

| | R | $R_1$ | $R_2$ | $R_8$ | $R_4$ | |
|---|---|---|---|---|---|---|
| 3.01 | H | F | Cl | H | H | |
| 3.02 | H | F | Cl | $CH_3$ | H | |
| 3.03 | H | F | Cl | H | $CH_3$ | |
| 3.04 | H | F | Cl | $CH_3$ | $CH_3$ | |
| 3.05 | H | F | Cl | H | H | |
| 3.06 | H | F | Cl | $CH_3$ | H | |
| 3.07 | H | F | Cl | H | $C_2H_5$ | |
| 3.08 | H | F | Cl | $CH_3$ | $C_2H_5$ | |
| 3.09 | H | F | Cl | H | $-CH_2-CH_2-OCH_3$ | |
| 3.10 | H | F | Cl | H | $-CH_2-CH=CH_2$ | |
| 3.11 | H | F | Cl | H | $-CH_2-C\equiv CH$ | |
| 3.12 | H | F | Cl | H | $-CH_2-CN$ | |
| 3.13 | H | F | Cl | H | $-CH_2-CH=CHCl$ | |
| 3.14 | H | F | Cl | H | $-CH_2-COOCH_3$ | |
| 3.15 | H | F | Cl | H | $-CH(CH_3)-COOCH_3$ | |
| 3.16 | H | F | Cl | H | 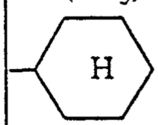 | |
| 3.17 | H | F | Cl | $CH_3$ | $-CH_2-CH_2-OCH_3$ | |
| 3.18 | H | F | Cl | $CH_3$ | $-CH_2-CH_2-SCH_3$ | |
| 3.19 | H | F | Cl | $CH_3$ | $-CH(CH_3)-CH_2-SCH_3$ | |
| 3.20 | H | F | Cl | $CH_3$ | $-CH_2-CH=CH_2$ | |
| 3.21 | H | F | Cl | $CH_3$ | $-CH_2-C\equiv CH$ | |
| 3.22 | H | F | Cl | $CH_3$ | $-CH_2-CN$ | |
| 3.23 | H | F | Cl | $CH_3$ | $-CH_2-COOCH_3$ | |
| 3.24 | H | F | Cl | $CH_3$ | $-CH(CH_3)-COOCH_3$ | |
| 3.25 | H | F | Cl | $CH_3$ | 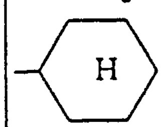 | |
| 3.26 | H | F | Cl | $CH_3$ | 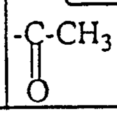 | |

Tabelle 3 (Fortsetzung)

| | R | $R_1$ | $R_2$ | $R_8$ | $R_4$ | |
|---|---|---|---|---|---|---|
| 3.27 | H | F | Cl | $CH_3$ | 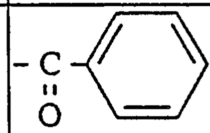 | |
| 3.28 | H | F | Cl | $CH_3$ | 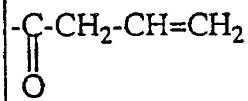 | |
| 3.29 | H | F | Cl | $CH_3$ | 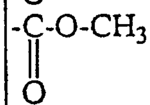 | |
| 3.30 | H | F | Cl | $CH_3$ | 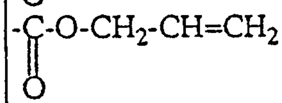 | |
| 3.31 | H | F | Cl | $CH_3$ | 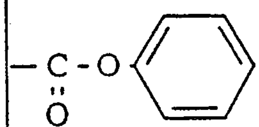 | |
| 3.32 | H | F | Cl | $CH_3$ | 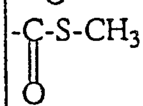 | |
| 3.33 | H | F | Cl | $CH_3$ | 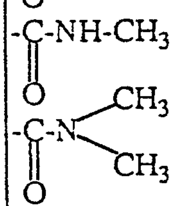 | |
| 3.34 | H | F | Cl | $CH_3$ | | |
| 3.35 | H | F | Cl | $CH_3$ | 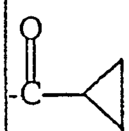 | |
| 3.36 | $CH_3$ | F | Cl | H | H | |
| 3.37 | $CH_3$ | F | Cl | $CH_3$ | H | |
| 3.38 | $CH_3$ | F | Cl | $CH_3$ | $CH_3$ | |
| 3.39 | $CH_3$ | H | Cl | H | H | |
| 3.40 | $CH_3$ | H | Cl | $CH_3$ | H | |
| 3.41 | $CH_3$ | H | Cl | $CH_3$ | $CH_3$ | |
| 3.42 | $CH_3$ | F | Cl | H | $C_2H_5$ | |
| 3.43 | $CH_3$ | F | Cl | $CH_3$ | $C_2H_5$ | |
| 3.44 | $CH_3$ | F | Cl | $CH_3$ | $CH_3$ | |
| 3.45 | $CH_3$ | F | Cl | H | $-CH_2-CH_2-CH_3$ | |
| 3.46 | $CH_3$ | F | Cl | H | $-CH_2-CH=CH_2$ | |
| 3.47 | $CH_3$ | F | Cl | $CH_3$ | $-CH_2-C\equiv CH$ | |
| 3.48 | $CH_3$ | F | Cl | H | $-CH_2-CN$ | |
| 3.49 | $CH_3$ | F | Cl | H | $-CH_2-CH=CHCl$ | |
| 3.50 | $CH_3$ | F | Cl | H | $-CH_2-COOCH_3$ | |
| 3.51 | $CH_3$ | F | Cl | H | $-CH(CH_3)COOCH_3$ | |

Tabelle 3 (Fortsetzung)

| | R | $R_1$ | $R_2$ | $R_8$ | $R_4$ | |
|---|---|---|---|---|---|---|
| 3.52 | $CH_3$ | F | Cl | H | 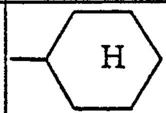 | |
| 3.53 | $CH_3$ | F | Cl | $CH_3$ | $-CH_2-CH_2-OCH_3$ | |
| 3.54 | $CH_3$ | F | Cl | $CH_3$ | $-CH_2-CH_2-SCH_3$ | |
| 3.55 | $CH_3$ | F | Cl | $CH_3$ | $-CH(CH_3)-SCH_3$ | |
| 3.56 | $CH_3$ | F | Cl | $CH_3$ | $-CH_2-CH=CH_2$ | |
| 3.57 | $CH_3$ | F | Cl | $CH_3$ | $-CH_2-C\equiv CH$ | |
| 3.58 | $CH_3$ | F | Cl | $CH_3$ | $-CH_2-CN$ | |
| 3.59 | $CH_3$ | F | Cl | $CH_3$ | $-CH_2-COOCH_3$ | |
| 3.60 | $CH_3$ | F | Cl | $CH_3$ | $-CH(CH_3)COOCH_3$ | |
| 3.61 | $CH_3$ | F | Cl | $CH_3$ | 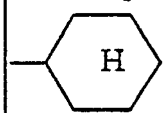 | |
| 3.62 | $CH_3$ | F | Cl | $CH_3$ | 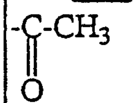 | |
| 3.63 | $CH_3$ | F | Cl | $CH_3$ | 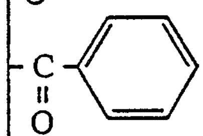 | |
| 3.64 | $CH_3$ | F | Cl | $CH_3$ | 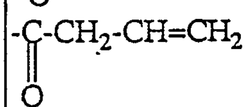 | |
| 3.65 | $CH_3$ | F | Cl | $CH_3$ | 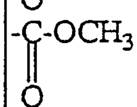 | |
| 3.66 | $CH_3$ | F | Cl | $CH_3$ | 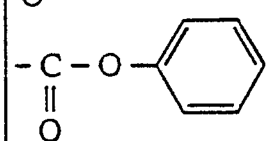 | |
| 3.67 | $CH_3$ | F | Cl | $CH_3$ | 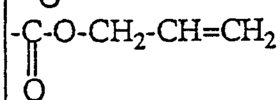 | |
| 3.68 | $CH_3$ | F | Cl | $CH_3$ | 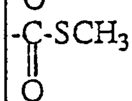 | |
| 3.69 | $CH_3$ | F | Cl | $CH_3$ | 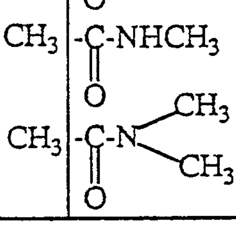 | |
| 3.70 | $CH_3$ | F | Cl | $CH_3$ | | |

Tabelle 3 (Fortsetzung)

| | R | $R_1$ | $R_2$ | $R_8$ | $R_4$ | |
|---|---|---|---|---|---|---|
| 3.71 | $CH_3$ | F | Br | $CH_3$ | $-CH_2-CH=CH_2$ | |
| 3.72 | $C_2H_5$ | F | Cl | H | $-CH_3$ | |
| 3.73 | $C_2H_5$ | F | Cl | H | $-CH_2-C\equiv CH$ | |
| 3.74 | $C_2H_5$ | F | Cl | H | $-CH_2COOCH_3$ | |
| 3.75 | $C_2H_5$ | F | Cl | H | $-CH(CH_3)-COOCH_3$ | |
| 3.76 | $C_2H_5$ | F | Cl | $-CH_3$ | $-CH_3$ | |
| 3.77 | $C_2H_5$ | F | Cl | $-CH_3$ | $-CH_2-C\equiv CH$ | |
| 3.78 | $C_2H_5$ | F | Cl | $-CH_3$ | $-CH_2COOCH_3$ | |
| 3.79 | $C_2H_5$ | F | Cl | $-CH_3$ | $-CH(CH_3)-COOCH_3$ | |
| 3.80 | $C_2H_5$ | F | Cl | $-CH_3$ | 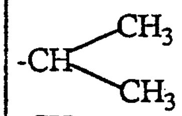 | |
| 3.81 | $C_3H_7$(i) | F | Cl | $-CH_3$ | $-CH_3$ | |
| 3.82 | $C_3H_7$(i) | F | Cl | $-CH_3$ | $-CH_2-C\equiv CH$ | |
| 3.83 | $C_3H_7$(i) | F | Cl | $-CH_3$ | $-CH_2COOCH_3$ | |
| 3.84 | $C_3H_7$(i) | F | Cl | $-CH_3$ | $-CH(CH_3)-COOCH_3$ | |
| 3.85 | $CH_3-O-CH_2-$ | F | Cl | $-CH_3$ | $-CH_3$ | |
| 3.86 | $CH_3-O-CH_2-$ | F | Cl | $-CH_3$ | $-CH_2-C\equiv CH$ | |
| 3.87 | $CH_2-O-CH_2-$ | F | Cl | $-CH_3$ | $-CH_2COOCH_3$ | |
| 3.88 | $CH_2-O-CH_2-$ | F | Cl | $-CH_3$ | $-CH(CH_3)-COOCH_3$ | |
| 3.89 | 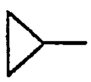 | F | Cl | $-CH_3$ | $-CH_3$ | |
| 3.90 | 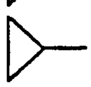 | F | Cl | $-CH_3$ | $-CH(CH_3)-COOCH_3$ | |
| 3.91 | 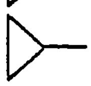 | F | Cl | $-CH_3$ | $-CH_2-CH=CHCl$ | |
| 3.92 | $CF_3$ | F | Cl | $-CH_3$ | $-CH_3$ | |
| 3.93 | 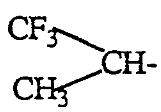 | F | Cl | $-CH_3$ | $-CH_3$ | |
| 3.94 | 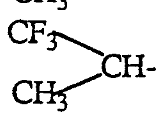 | F | Cl | $-CH_3$ | $C_2H_5$ | |

Verbindungen der Formel

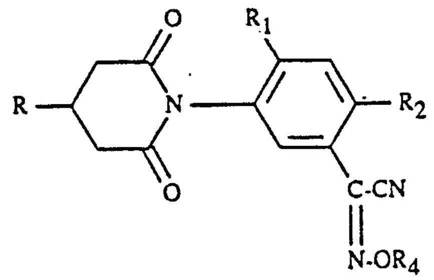

(Id),

Tabelle 4

| | R | $R_1$ | $R_2$ | $R_4$ | |
|---|---|---|---|---|---|
| 4.01 | $CH_3$ | H | Cl | $CH_3$ | |
| 4.02 | $CH_3$ | H | Cl | $-CH_2-CH=CH_2$ | |
| 4.03 | $CH_3$ | H | Cl | $-CH_2-COOCH_3$ | |
| 4.04 | $CH_3$ | H | Cl | $-CH(CH_3)-COOCH_3$ | |
| 4.05 | $CH_3$ | H | Cl | $-CH_2C\equiv CH$ | |
| 4.06 | $CH_3$ | H | Cl | $-CH_2-COOC_5H_{11}$ | |
| 4.07 | $CH_3$ | H | Cl | $-CH_2-COSCH_3$ | |
| 4.08 | $CH_3$ | H | Cl | $-CH_2-COSC_4H_9$ | |
| 4.09 | $CH_3$ | F | Cl | $CH_3$ | |
| 4.10 | $CH_3$ | F | Cl | $C_2H_5$ | |
| 4.11 | $CH_3$ | F | Cl | 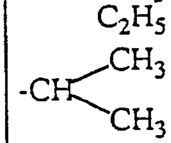 | |
| 4.12 | $CH_3$ | F | Cl | $-CH_2-CH=CH_2$ | |
| 4.13 | $CH_3$ | F | Cl | $-CH_2-C\equiv CH$ | |
| 4.14 | $CH_3$ | F | Cl | $-CH_2-COOCH_3$ | |
| 4.15 | $CH_3$ | F | Cl | 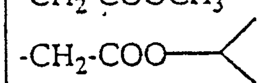 | |
| 4.16 | $CH_3$ | F | Cl | $-CH_2-COOC_5H_{11}$ | |
| 4.17 | $CH_3$ | F | Cl | $-CH_2(CH_3)COOCH_3$ | |
| 4.18 | $CH_3$ | F | Cl | $-CH_2-COSCH_3$ | |
| 4.19 | $CH_3$ | F | Cl | 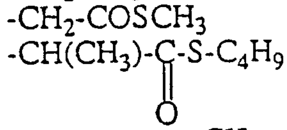 | |
| 4.20 | $CH_3$ | F | Cl | 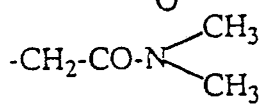 | |
| 4.21 | $CH_3$ | F | Cl | 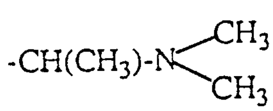 | |
| 4.22 | $CH_3$ | F | Cl | $-CH_2-CH_2-OCH_3$ | |
| 4.23 | $CH_3$ | F | Cl | $-CH(CH_3)-SCH_3$ | |

Tabelle 4 (Fortsetzung)

| | R | $R_1$ | $R_2$ | $R_4$ | |
|---|---|---|---|---|---|
| 4.24 | $CH_3$ | F | Cl | $-CH_2-CN$ | |
| 4.25 | $CH_3$ | F | Cl | $-CH_2-CH=CHCl$ | |
| 4.26 | $CH_3$ | F | Cl | $-CH_2-CHCl=CH_2$ | |
| 4.27 | $CH_3$ | F | Cl | 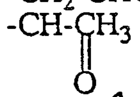 | |
| 4.28 | $CH_3$ | F | Cl | 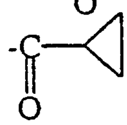 | |
| 4.29 | $CH_3$ | F | Cl | 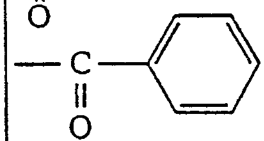 | |
| 4.30 | $CH_3$ | F | Cl | 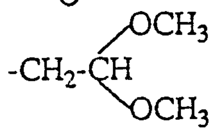 | |
| 4.31 | $C_2H_5$ | F | Cl | $CH_3$ | |
| 4.32 | $C_2H_5$ | F | Cl | $-CH_2-C\equiv CH$ | |
| 4.33 | $C_2H_5$ | F | Cl | $-CH_2-CH=CH_2$ | |
| 4.34 | $C_2H_5$ | F | Cl | $-CH_2-COOCH_3$ | |
| 4.35 | $C_2H_5$ | F | Cl | 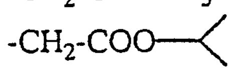 | |
| 4.36 | $C_2H_5$ | F | Cl | $-CH_2-COOC_5H_{11}$ | |
| 4.37 | $C_2H_5$ | F | Cl | $-CH(CH_3)-COOCH_3$ | |
| 4.38 | $C_2H_5$ | F | Cl | 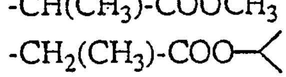 | |
| 4.39 | $C_3H_7(i)$ | F | Cl | $-CH_3$ | |
| 4.40 | $C_3H_7(i)$ | F | Cl | $-CH_2-COOCH_3$ | |
| 4.41 | $C_3H_7(i)$ | F | Cl | $-CH_2-COOC_5H_{11}$ | |
| 4.42 | $C_3H_7(i)$ | F | Cl | $-CH_2-CHCl=CH_2$ | |
| 4.43 | $C_3H_7(i)$ | F | Cl | $-CH_2-C\equiv CH$ | |
| 4.44 | $C_3H_7(i)$ | F | Cl | 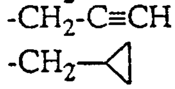 | |
| 4.45 | $C_3H_7(i)$ | F | Cl | $-CH(CH_3)-SCH_3$ | |
| 4.46 | $CF_3$ | F | Cl | $-CH_3$ | |
| 4.47 | 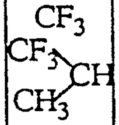 | F | Cl | $-CH_3$ | |

Verbindungen der Formel

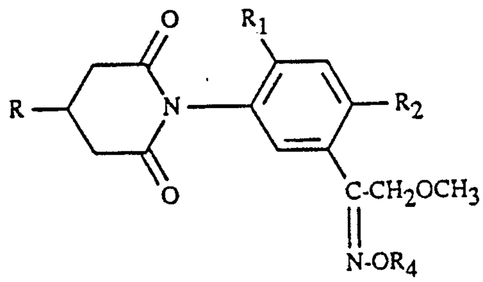

(Ie)

Tabelle 5

| | R | $R_1$ | $R_2$ | $R_4$ | |
|---|---|---|---|---|---|
| 5.01 | $CH_3$ | H | Cl | $CH_3$ | |
| 5.02 | $CH_3$ | H | Cl | $-CH_2-CH=CH_2$ | |
| 5.03 | $CH_3$ | H | Cl | $-CH_2-C\equiv CH$ | |
| 5.04 | $CH_3$ | H | Cl | $-CH_2-COOCH_3$ | |
| 5.05 | $CH_3$ | H | Cl | $-CH_2-COOC_5H_{11}$ | |
| 5.06 | $CH_3$ | H | Cl | $-CH(CH_3)-COOCH_3$ | |
| 5.07 | $CH_3$ | F | Cl | $CH_3$ | |
| 5.08 | $CH_3$ | F | Cl | 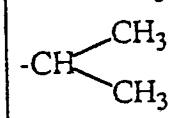 | |
| 5.09 | $CH_3$ | F | Cl | $-CH_2-C\equiv CH$ | |
| 5.10 | $CH_3$ | F | Cl | $-CH_2-COOCH_3$ | |
| 5.11 | $CH_3$ | F | Cl | $-CH_2-COOC_5H_{11}$ | |
| 5.12 | $CH_3$ | F | Cl | $-CH_2-COSCH_3$ | |
| 5.13 | $CH_3$ | F | Cl | $-CH(CH_3)-COOCH_3$ | |
| 5.14 | $CH_3$ | F | Cl | $-CH(CH_3)-COOC_2H_5$ | |
| 5.15 | $CH_3$ | F | Cl | $-CH(CH_3)-COO-$ 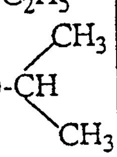 | |
| 5.16 | $CH_3$ | F | Cl | $-CH(CH_3)-SCH_3$ | |
| 5.17 | $CH_3$ | F | Cl | $-CH(CH_3)COSC_4H_9$ | |
| 5.18 | $C_3H_7$(i) | F | Cl | $CH_3$ | |
| 5.19 | $CF_3$ | F | Cl | $CH_3$ | |
| 5.20 | 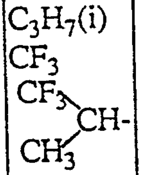 | F | Cl | $CH_3$ | |

Verbindungen der Formel

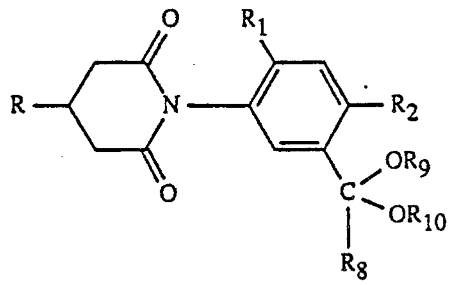

(If)

Tabelle 6

| | R | $R_1$ | $R_2$ | $R_8$ | $R_9$ | $R_{10}$ | |
|---|---|---|---|---|---|---|---|
| 6.01 | $CH_3$ | F | Cl | $CH_3$ | $CH_3$ | $CH_3$ | |
| 6.02 | $CH_3$ | F | Cl | $CH_3$ | $C_2H_5$ | $C_2H_5$ | |
| 6.03 | $CH_3$ | F | Cl | H | $CH_3$ | $CH_3$ | |
| 6.04 | $CH_3$ | F | Cl | $CH_2$-O-$CH_3$ | $CH_3$ | $CH_3$ | |
| 6.05 | $C_2H_5$ | F | Cl | $CH_3$ | $CH_3$ | $CH_3$ | |
| 6.06 | $CH_3$ | H | Cl | $CH_3$ | $CH_3$ | $CH_3$ | |
| 6.07 | $C_3H_7$(i) | F | Cl | $CH_3$ | $CH_3$ | $CH_3$ | |
| 6.08 | $CF_3$ | F | Cl | $CH_3$ | $CH_3$ | $CH_3$ | |
| 6.09 | 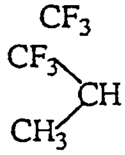 | F | Cl | $CH_3$ | $CH_3$ | $CH_3$ | |

Verbindungen der Formel

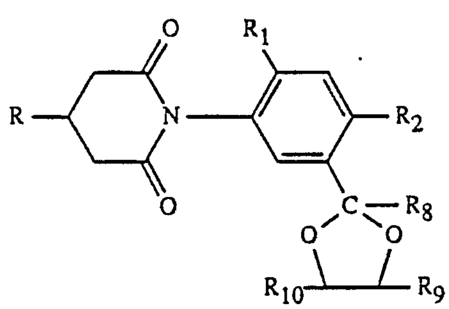

Tabelle 7

| | R | $R_1$ | $R_2$ | $R_8$ | $R_9$ | $R_{10}$ | |
|---|---|---|---|---|---|---|---|
| 7.01 | $CH_3$ | H | Cl | $CH_3$ | H | H | |
| 7.02 | $CH_3$ | H | Cl | $CH_3$ | $CH_3$ | H | |
| 7.03 | $CH_3$ | H | Cl | $CH_3$ | $C_2H_5$ | H | |
| 7.04 | $CH_3$ | H | Cl | $CH_3$ | $C_3H_7$ | H | |
| 7.05 | $CH_3$ | H | Cl | $CH_3$ | $CH_3$ | $CH_3$ | |
| 7.06 | $CH_3$ | F | Cl | $CH_3$ | H | H | |
| 7.07 | $CH_3$ | F | Cl | $CH_3$ | $CH_3$ | H | |
| 7.08 | $CH_3$ | F | Cl | $CH_3$ | $C_2H_5$ | H | |
| 7.09 | $CH_3$ | F | Cl | $CH_3$ | $CH_3$ | $CH_3$ | |
| 7.10 | $CH_3$ | F | Cl | $CH_3$ | $CH_3$ | $C_2H_5$ | |
| 7.11 | $CF_3$ | F | Cl | $CH_3$ | $CH_3$ | $CH_3$ | |
| 7.12 | $C_3H_7$(i) | F | Cl | $CH_3$ | $CH_3$ | $CH_3$ | |
| 7.13 | 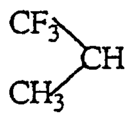 | F | Cl | $CH_3$ | $CH_3$ | $CH_3$ | |

Verbindungen der Formel

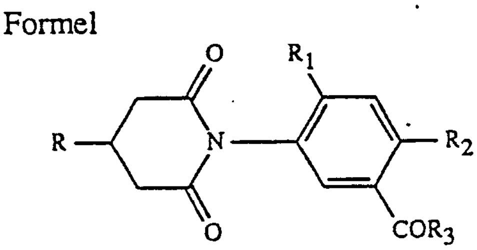

(Ib)

Tabelle 8

| | R | $R_1$ | $R_2$ | $R_3$ | |
|---|---|---|---|---|---|
| 8.01 | $CH_3$ | H | Cl | OH | |
| 8.02 | $CH_3$ | H | Cl | $OCH_3$ | |
| 8.03 | $CH_3$ | H | Cl | $OC_2H_5$ | |
| 8.04 | $CH_3$ | H | Cl | $O-C_3H_7$ | |
| 8.05 | $CH_3$ | H | Cl | 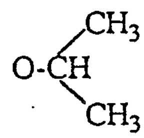 | |
| 8.06 | $CH_3$ | H | Cl | $OC_5H_{11}$ | |
| 8.07 | $CH_3$ | H | Cl | $OCH_2-CH_2-OCH_3$ | |
| 8.08 | $CH_3$ | H | Cl | $OCH(CH_3)-SCH_3$ | |
| 8.09 | $CH_3$ | H | Cl | $OCH(CH_3)-S-C_2H_5$ | |
| 8.10 | $CH_3$ | H | Cl | 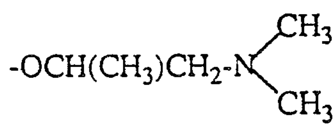 | |
| 8.11 | $CH_3$ | H | Cl | $OCH-CH=CH_2$ | |
| 8.12 | $CH_3$ | H | Cl | $OCH_2-C \equiv CH$ | |
| 8.13 | $CH_3$ | H | Cl | 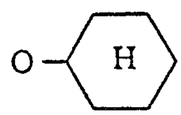 | |
| 8.14 | $CH_3$ | H | Cl | 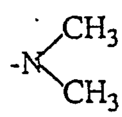 | |
| 8.15 | $CH_3$ | H | Cl | 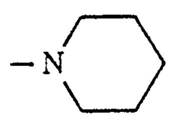 | |
| 8.16 | $CH_3$ | H | Cl | 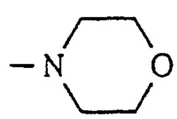 | |
| 8.17 | | H | | 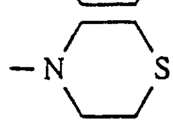 | |
| 8.18 | $CH_3$ | H | Cl | $-N-(CH_2-CH=CH_2)_2$ | |

Tabelle 8 (Fortsetzung)

| | R | $R_1$ | $R_2$ | $R_3$ | |
|---|---|---|---|---|---|
| 8.19 | $CH_3$ | H | Cl | $-SCH_3$ | |
| 8.20 | $CH_3$ | H | Cl | $-C_3H_7$ | |
| 8.21 | $CH_3$ | H | Cl | $-SCH_2-CH=CH_2$ | |
| 8.22 | $CH_3$ | H | Cl | $-SCH_2-COOCH_3$ | |
| 8.23 | $CH_3$ | H | Cl | $-SCH_2-COOC_2H_5$ | |
| 8.24 | $CH_3$ | H | Cl | $-SCH_2-COOC_5H_{11}$ | |
| 8.25 | $CH_3$ | H | Cl | $-SCH(CH_3)-COOCH_3$ | |
| 8.26 | $CH_3$ | H | Cl | $-SCH(CH_3)-COOC_2H_5$ | |
| 8.27 | $CH_3$ | H | Cl | $-SCH(CH_3)-COOC_3H_7$ | |
| 8.28 | $CH_3$ | H | Cl | $-SCH_2-CH_2-COOCH_3$ | |
| 8.29 | $CH_3$ | H | Cl | $-SCH_2-COOCH_2-CH_2-OCH_3$ | |
| 8.30 | $CH_3$ | H | Cl | $-OCH_2-CH_2-COOCH_3$ | |
| 8.31 | $CH_3$ | H | Cl | $-OCH(CH_3)-COOCH_3$ | |
| 8.32 | $CH_3$ | H | Cl | $-OCH_2-COOC_5H_{11}$ | |
| 8.33 | $CH_3$ | H | Cl | $-OCH_2-COS-CH_3$ | |
| 8.34 | $CH_3$ | H | Cl | $-OCH_2-CN$ | |
| 8.35 | $CH_3$ | F | Cl | $-OH$ | |
| 8.36 | $CH_3$ | F | Cl | $-OCH_3$ | Smp. 116-118° |
| 8.37 | $CH_3$ | F | Cl | $-OC_2H_5$ | $n_D^{25}$ 1.5336 |
| 8.38 | $CH_3$ | F | Cl | $-OC_3H_7$(iso) | $n_D^{22}$ 1.5169 |
| 8.39 | $CH_3$ | F | Cl | $-OC_3H_7$(n) | |
| 8.40 | $CH_3$ | F | Cl | $OC_5H_{11}$ | |
| 8.41 | $CH_3$ | F | Cl | $-OCH_2-CH_2-OCH_3$ | Smp. 84-86° |
| 8.42 | $CH_3$ | F | Cl | $-OCH(CH_3)-SCH_3$ | |
| 8.43 | $CH_3$ | F | Cl | $-OCH(CH_3)-S-C_2H_5$ | |
| 8.44 | $CH_3$ | F | Cl | $-OCH(CH_3)--CH_2-$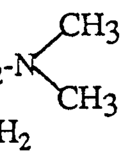 | |
| 8.45 | $CH_3$ | F | Cl | $-OCH_2-CH=CH_2$ | |
| 8.46 | $CH_3$ | F | Cl | $-OCH_2-C\equiv CH$ | |
| 8.47 | $CH_3$ | F | Cl | 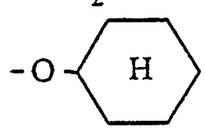 | |
| 8.48 | $CH_3$ | F | Cl | 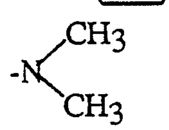 | |
| 8.49 | $CH_3$ | F | Cl | 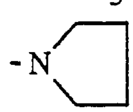 | |
| 8.50 | $CH_3$ | F | Cl | 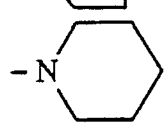 | |

Tabelle 8 (Fortsetzung)

| | R | $R_1$ | $R_2$ | $R_3$ | |
|---|---|---|---|---|---|
| 8.51 | $CH_3$ | F | Cl | 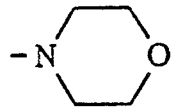 | |
| 8.52 | $CH_3$ | F | Cl | 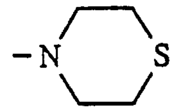 | |
| 8.53 | $CH_3$ | F | Cl | $-SCH_3$ | |
| 8.54 | $CH_3$ | F | Cl | $-S-C_3H_7$(iso) | $n_D^{20}$ 1.5378 |
| 8.55 | $CH_3$ | F | Cl | $-SCH_2-CH=CH_2$ | |
| 8.56 | $CH_3$ | F | Cl | $-SCH_2-COOCH_3$ | $n_D^{22}$ 1.5581 |
| 8.57 | $CH_3$ | F | Cl | $-SCH_2-COOC_2H_5$ | $n_D^{21}$ 1.5429 |
| 8.58 | $CH_3$ | F | Cl | $-SCH_2-COOC_5H_{11}$ | |
| 8.59 | $CH_3$ | F | Cl | $-SCH(CH_3)-COOCH_3$ | $n_D^{22}$ 1.5526 |
| 8.60 | $CH_3$ | F | Cl | $-SCH(CH_3)-COOC_2H_5$ | $n_D^{22}$ 1.5353 |
| 8.61 | $CH_3$ | F | Cl | $-SCH(CH_3)-COOC_3H_7$ | |
| 8.62 | $CH_3$ | F | Cl | $-SCH_2-CH_2-COOCH_3$ | |
| 8.63 | $CH_3$ | F | Cl | $-SCH_2-COOCH_2-CH_2-OCH_3$ | |
| 8.64 | $CH_3$ | F | Cl | $-OCH_2-COOCH_3$ | |
| 8.65 | $CH_3$ | F | Cl | $-OCH(CH_3)-COOCH_3$ | |
| 8.66 | $CH_3$ | F | Cl | $-OCH_2-COOC_5H_{11}$ | |
| 8.67 | $CH_3$ | F | Cl | $-OCH_2-COSCH_3$ | |
| 8.68 | $CH_3$ | F | Cl | $-OCH_2-CN$ | |
| 8.69 | $C_3H_7$(i) | F | Cl | -OH | Smp. 205-207°C |
| 8.70 | $C_3H_7$(i) | F | Cl | $-OCH_3$ | |
| 8.71 | $C_3H_7$(i) | F | Cl | $-OC_2H_5$ | |
| 8.72 | $C_3H_7$(i) | F | Cl | $-OC_3H_7$ | |
| 8.73 | $C_3H_7$(i) | F | Cl | $-OC_3H_7$(i) | $n_D^{23}$ 1.5187 |
| 8.74 | $C_3H_7$(i) | F | Cl | $-OC_5H_{11}$ | |
| 8.75 | $C_3H_7$(i) | F | Cl | $-O-CH_2-CH_2-OCH_3$ | |
| 8.76 | $C_3H_7$(i) | F | Cl | $-O-CH(CH_3)-SCH_3$ | |
| 8.77 | $C_3H_7$(i) | F | Cl | $-O-CH(CH_3)-S-C_2H_5$ | |
| 8.78 | $C_3H_7$(i) | F | Cl | $-O-CH(CH_3)-CH_2-$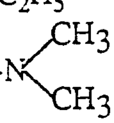 | |
| 8.79 | $C_3H_7$(i) | F | Cl | $-O-CH_2-CH=CH_2$ | |
| 8.80 | $C_3H_7$(i) | F | Cl | $-O-CH_2-C\equiv CH$ | |
| 8.81 | $C_3H_7$(i) | F | Cl | 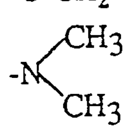 | |
| 8.82 | $C_3H_7$(i) | F | Cl | 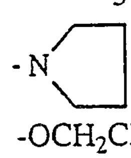 | |
| 8.83 | $CH_3$ | F | Cl | $-OCH_2CH_2SCH_3$ | Harz |
| 8.84 | $CH_3$ | F | Cl | $-OCH_2CH_2SO_2CH_3$ | Harz |

Tabelle 8 (Fortsetzung)

| | R | $R_1$ | $R_2$ | $R_3$ | |
|---|---|---|---|---|---|
| 8.83 | $C_3H_7$(i) | F | Cl | 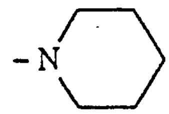 | |
| 8.84 | $C_3H_7$(i) | F | Cl | 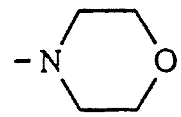 | |
| 8.85 | $C_3H_7$(i) | F | Cl | 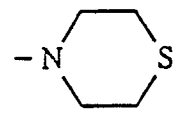 | |
| 8.86 | $C_3H_7$(i) | F | Cl | $-SCH_3$ | |
| 8.87 | $C_3H_7$(i) | F | Cl | $-S-C_3H_7$ | |
| 8.88 | $C_3H_7$(i) | F | Cl | $-SCH_2-CH=CH_2$ | |
| 8.89 | $C_3H_7$(i) | F | Cl | $-SCH_2-COOCH_3$ | $n_D^{23}$ 1.5516 |
| 8.90 | $C_3H_7$(i) | F | Cl | $-SCH_2-COOC_2H_5$ | $n_D^{21}$ 1.5428 |
| 8.91 | $C_3H_7$(i) | F | Cl | $-SCH(CH_3)-COOCH_3$ | $n_D^{23}$ 1.5463 |
| 8.92 | $C_3H_7$(i) | F | Cl | $-SCH(CH_3)-COOC_2H_5$ | $n_D^{23.5}$ 1.5438 |
| 8.93 | $C_3H_7$(i) | F | Cl | $-SCH(CH_3)-COOC_3H_7$ | |
| 8.94 | $C_3H_7$(i) | F | Cl | $-SCH_2-CH_2-COOCH_3$ | |
| 8.95 | $C_3H_7$(i) | F | Cl | $-SCH_2-COOCH_2-CH_2-OCH_3$ | |
| 8.96 | $C_3H_7$(i) | F | Cl | $-OCH_2-COOCH_3$ | |
| 8.97 | $C_3H_7$(i) | F | Cl | $-OCH(CH_3)-COOCH_3$ | |
| 8.98 | $C_3H_7$(i) | F | Cl | $-OCH_2-COOC_5H_{11}$ | |
| 8.99 | $C_3H_7$(i) | F | Cl | $-OCH_2-COSCH_3$ | |
| 8.100 | $C_3H_7$(i) | F | Cl | $-OCH_2-CN$ | |
| 8.101 | $CH_3$ | F | CN | 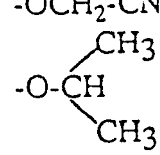 | |
| 8.102 | $CH_3$ | F | $NO_2$ | -OH | |
| 8.103 | $CH_3$ | F | $NO_2$ | 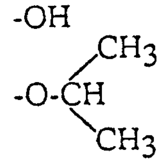 | |
| 8.104 | $CH_3$ | F | Br | -OH | |
| 8.105 | $CH_3$ | F | Br | 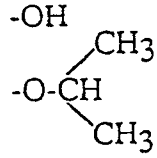 | |
| 8.106 | $C_2H_5$ | F | Br | $-OCH_2-COOCH_3$ | |
| 8.107 | $C_2H_5$ | F | Br | $-SCH_2-COOCH_3$ | |
| 8.108 | $C_2H_5$ | F | Br | $-SCH(CH_3)-COOCH_3$ | |
| 8.109 | $C_2H_5$ | F | Br | $-SCH(CH_3)-COOC_2H_5$ | |
| 8.110 | $C_2H_5$ | F | Br | $-O-CH_2-COOC_5H_{11}$ | |
| 8.111 | $C_2H_5$ | F | Cl | $-OCH_3$ | |

Tabelle 8 (Fortsetzung)

| | R | $R_1$ | $R_2$ | $R_3$ | |
|---|---|---|---|---|---|
| 8.112 | $CH_3$ | F | Cl | $-OC_4H_9$(sec) | $n_D^{25}$ 1.5221 |
| 8.113 | $CH_3$ | F | Cl | $-OC_4H_9$(iso) | $n_D^{21}$ 1.5184 |
| 8.114 | $CH_3$ | F | Cl | $-OCH(CH_3)CH_2-SC_2H_5$ | $n_D^{22}$ 1.5339 |
| 8.115 | $CH_3$ | F | Cl | $-OCH(CH_3)CH_2-SCH_3$ | $n_D^{22}$ 1.5387 |
| 8.116 | $CH_3$ | F | Cl | $-OCH(CH_3)CH_2-S-C_3H_7$-n | $n_D^{21}$ 1.5273 |
| 8.117 | $CH_3$ | F | Cl | $-OCH(CH_3)CH_2-S-CH(CH_3)_2$ | $n_D^{22}$ 1.5252 |
| 8.118 | $CH_3$ | F | Cl | $-OCH(CH_3)CH_2-S-C_5H_{11}$-n | $n_D^{22}$ 1.5153 |
| 8.119 | $C_2H_5$ | F | Cl | $-OCH_3$ | $n_D^{23}$ 1.5349 |
| 8.120 | $C_3H_7$(i) | F | Cl | $-OCH(CH_3)CH_2SCH_3$ | $n_D^{21}$ 1.5292 |
| 8.121 | $C_3H_7$(i) | F | Cl | $-OCH(CH_3)CH_2SC_2H_5$ | $n_D^{21}$ 1.5339 |
| 8.122 | $C_3H_7$(i) | F | Cl | $-OCH(CH_3)CH_2SC_3H_7$(n) | $n_D^{21}$ 1.5221 |
| 8.123 | $C_3H_7$(i) | F | Cl | $-OCH(CH_3)CH_2SC_3H_7$(i) | $n_D^{21}$ 1.5247 |
| 8.124 | $C_3H_7$(i) | F | Cl | $-OCH(CH_3)CH_2SC_4H_9$(n) | $n_D^{21}$ 1.5178 |
| 8.125 | $C_3H_7$(i) | F | Cl | $-OCH(CH_3)CH_2SC_5H_{11}$(n) | $n_D^{21}$ 1.5209 |
| 8.126 | $C_3H_7$(i) | F | Cl | $-OCH(CH_3)CH_2SC_4H_9$(s) | $n_D^{21}$ 1.5219 |
| 8.127 | $CF_3$ | F | Cl | $-OCH(CH_3)CH_2S-CH_3$ | |
| 8.128 | $CF_3CF_2$ | F | Cl | $-OCH(CH_3)CH_2S-C_2H_5$ | |
| 8.129 | $CF_3$ | F | Cl | -OH | |
| 8.130 | $CF_3$ | F | Cl | $-OCH_3$ | |
| 8.131 | $CF_3$ | F | Cl | $-OC_2H_5$ | |
| 8.132 | $CF_3$ | F | Cl | 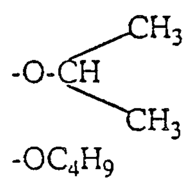 | |
| 8.133 | $CF_3$ | F | Cl | $-OC_4H_9$ | |
| 8.134 | $CF_3$ | F | Cl | 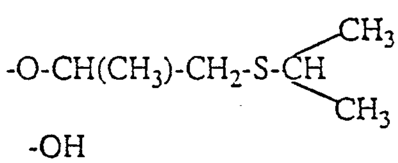 | |
| 8.135 | $CH_3$ | F | Br | -OH | |
| 8.136 | $CH_3$ | F | Br | $-OCH_3$ | |
| 8.137 | $CH_3$ | F | Br | $-OC_2H_5$ | |
| 8.138 | $CH_3$ | F | Br | 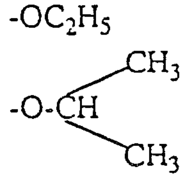 | |
| 8.139 | $CH_3$ | F | Br | $-OCH_2-CH_2-O-CH_3$ | |
| 8.140 | $CH_3$ | F | Br | $-OCH_2-CH_2-S-CH_3$ | |
| 8.141 | $CH_3$ | F | Br | $-OCH_2-CH=CH_2$ | |

Tabelle 8 (Fortsetzung)

| | R | $R_1$ | $R_2$ | $R_3$ | |
|---|---|---|---|---|---|
| 8.141 | $CH_3$ | F | Br | $-O-CH_2-CH=CH_2$ | |
| 8.142 | $CH_3$ | F | Br | $-O-CH_2-C\equiv CH$ | |
| 8.143 | $CH_3$ | F | Br | $-S-C_3H_7$ | |
| 8.144 | $CH_3$ | F | Br | $-S-CH_2-COOCH_3$ | |
| 8.145 | $CH_3$ | F | Br | $-S-CH(CH_3)COOCH_3$ | |
| 8.146 | $CH_3$ | F | Br | $-O-CH(CH_3)CH_2-S-CH_3$ | |
| 8.147 | $CH_3$ | F | Br | $-O-CH(CH_3)CH_2-S-C_2H_5$ | |
| 8.148 | $CH_3$ | F | Br | $-O-CH(CH_3)CH_2-S-C_3H_7(i)$ | |
| 8.149 | $CH_3$ | F | Br | 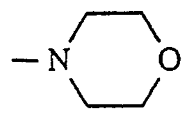 | |
| 8.150 | $CH_3$ | F | Br | 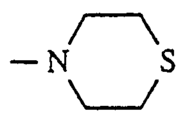 | |
| 8.151 | $CH_3$ | F | Br | 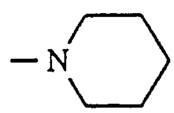 | |
| 8.152 | $C_3H_7(i)$ | F | Br | $-OH$ | |
| 8.153 | $C_3H_7(i)$ | F | Br | $-OCH_3$ | |
| 8.154 | $C_3H_7(i)$ | F | Br | $-OC_2H_5$ | |
| 8.155 | $C_3H_7(i)$ | F | Br | 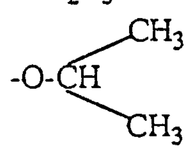 | |
| 8.156 | $C_3H_7(i)$ | F | Br | $-OCH_2-CH_2-O-CH_3$ | |
| 8.157 | $C_3H_7(i)$ | F | Br | $-O-CH_2-CH_2-O-CH_3$ | |
| 8.158 | $C_3H_7(i)$ | F | Br | $-O-CH_2-CH_2-S-CH_3$ | |
| 8.159 | $C_3H_7(i)$ | F | Br | $-O-CH_2-CH=CH_2$ | |
| 8.160 | $C_3H_7(i)$ | F | Br | $-O-CH_2-C\equiv CH$ | |
| 8.161 | $C_3H_7(i)$ | F | Br | $-S-C_3H_7$ | |
| 8.162 | $C_3H_7(i)$ | F | Br | $-S-CH_2-COOCH_3$ | |
| 8.163 | $C_3H_7(i)$ | F | Br | $-S-CH_2(CH_3)COOCH_3$ | |
| 8.164 | $C_3H_7(i)$ | F | Br | $-O-CH(CH_3)-CH_2-S-CH_3$ | |
| 8.165 | $C_3H_7(i)$ | F | Br | $-O-CH(CH_3)-CH_2-S-C_2H_5$ | |
| 8.166 | $C_3H_7(i)$ | F | Br | $-O-CH(CH_3)-CH_2-S-C_3H_7(i)$ | |

Tabelle 8 (Fortsetzung)

| | R | $R_1$ | $R_2$ | $R_3$ | |
|---|---|---|---|---|---|
| 8.167 | $C_3H_7$(i) | F | Br | 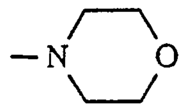 | |
| 8.168 | $C_3H_7$(i) | F | Br | 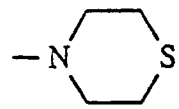 | |
| 8.169 | $C_3H_7$(i) | F | Br | 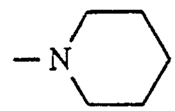 | |
| 8.170 | $CF_3$ | F | Br | -OH | |
| 8.171 | $CF_3$ | F | Br | $-OCH_3$ | |
| 8.172 | $CF_3$ | F | Br | $-O-C_3H_7$(i) | |
| 8.173 | $CF_3$ | F | Br | $-O-CH_2-CH_2-O-CH_3$ | |
| 8.174 | $CF_3$ | F | Br | $-O-CH_2-CH_2-S-CH_3$ | |
| 8.175 | $CF_3$ | F | Br | $-O-CH_2-CH=CH_2$ | |
| 8.176 | $CF_3$ | F | Br | $-O-CH_2-C\equiv CH$ | |
| 8.177 | $CF_3$ | F | Br | $-S-C_3H_7$ | |
| 8.178 | $CF_3$ | F | Br | $-S-CH_2-COOCH_3$ | |
| 8.179 | $CF_3$ | F | Br | $-S-CH(CH_3)COOCH_3$ | |
| 8.180 | $CF_3$ | F | Br | $-O-CH(CH_3)CH_2-S-CH_3$ | |
| 8.181 | $CF_3$ | F | Br | $-O-CH(CH_3)CH_2-S-C_2H_5$ | |
| 8.182 | $CF_3$ | F | Br | $-O-CH(CH_3)CH_2-S-C_3H_7$(i) | |
| 8.183 | $CF_3$ | F | Br | 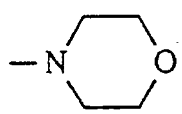 | |
| 8.184 | $CF_3$ | F | Br | 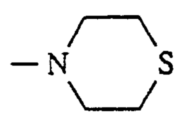 | |
| 8.185 | $CF_3$ | F | Br | 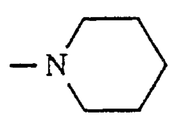 | |
| 8.186 | 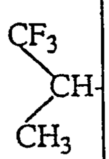 | F | Cl | -OH | |

Tabelle 8 (Fortsetzung)

| | R | $R_1$ | $R_2$ | $R_3$ | |
|---|---|---|---|---|---|
| 8.188 | 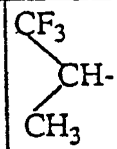 | F | Cl | $-OCH_3$ | |
| 8.189 | 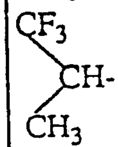 | F | Cl | $-O-C_3H_7(i)$ | |
| 8.190 | 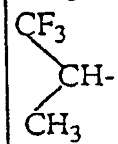 | F | Cl | $-O-CH_2-CH_2-O-CH_3$ | |
| 8.191 | 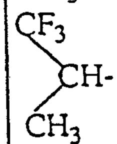 | F | Cl | $-O-CH_2-CH_2-S-CH_3$ | |
| 8.192 | 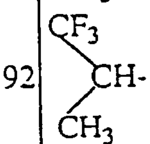 | F | Cl | $-O-CH_2-CH=CH_2$ | |
| 8.193 | 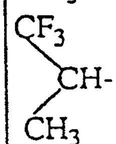 | F | Cl | $-O-CH_2-C\equiv CH$ | |
| 8.194 | 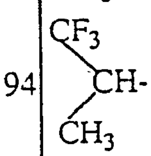 | F | Cl | $-S-C_3H_7$ | |
| 8.195 | 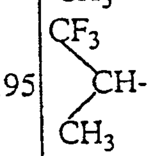 | F | Cl | $-S-CH_2-COOCH_3$ | |
| 8.196 | 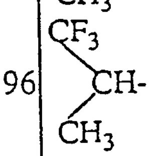 | F | Cl | $-S-CH(CH_3)COOCH_3$ | |

Tabelle 8 (Fortsetzung)

| | R | $R_1$ | $R_2$ | $R_3$ | |
|---|---|---|---|---|---|
| 8.197 | 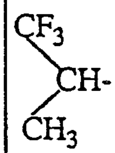 | F | Cl | $-O-CH(CH_3)CH_2-S-CH_3$ | |
| 8.198 | 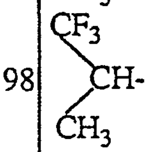 | F | Cl | $-O-CH(CH_3)CH_2-S-C_2H_5$ | |
| 8.199 | 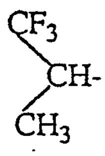 | F | Cl | $-O-CH(CH_3)CH_2-S-C_3H_7(i)$ | |
| 8.200 | 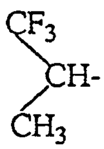 | F | Cl | 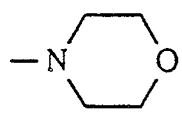 | |
| 8.201 | 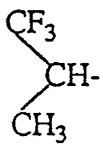 | F | Cl | 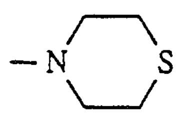 | |
| 8.202 | 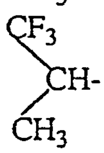 | F | Cl | 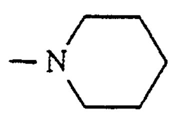 | |

Verbindungen der Formel

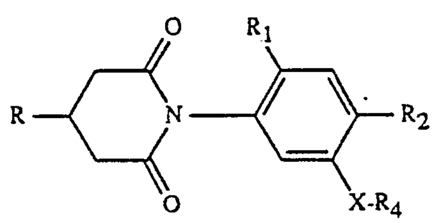

(Ih)

Tabelle 9

| | R | $R_1$ | $R_2$ | X | $R_4$ | |
|---|---|---|---|---|---|---|
| 9.01 | H | F | Cl | O | $CH_2$-C≡CH | Smp. 206-209°C |
| 9.02 | H | F | Cl | O | 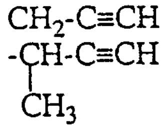 | |
| 9.03 | H | F | Cl | O | -$CH_2$-$COOC_5H_{11}$ | |
| 9.04 | H | F | Cl | O | -$CH_2$-$COOCH_3$ | |
| 9.05 | H | F | Cl | O | -$CH_2$-$COOC_2H_5$ | |
| 9.06 | H | F | Cl | O | -$CH_2$-$COOCH_3$ | |
| 9.07 | H | F | Cl | O | -$CH_3$ | |
| 9.08 | H | F | Cl | O | -$C_2H_5$ | |
| 9.09 | H | F | Cl | O | -$C_3H_7$ | |
| 9.10 | H | F | Cl | O | 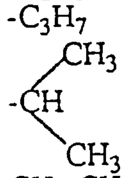 | Smp. (92°) 97-98°C |
| 9.11 | H | F | Cl | O | $CH_2$-CH=$CH_2$ | |
| 9.12 | H | F | Cl | O | -$CH_2$-CHCl=$CH_2$ | |
| 9.13 | H | F | Cl | O | -$CH_2$-CH=CHCl | |
| 9.14 | H | F | Cl | S | -$CH_2$-C≡CH | |
| 9.15 | H | F | Cl | S | 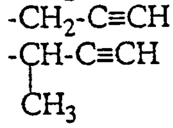 | |
| 9.16 | H | F | Cl | S | -$CH_2$-$COOC_5H_{11}$ | |
| 9.17 | H | F | Cl | S | -$CH_2$-$COOCH_3$ | |
| 9.18 | H | F | Cl | S | -$CH_2$-$COOC_2H_5$ | |
| 9.19 | H | F | Cl | S | -$CH_2(CH_3)$-$COOCH_3$ | |
| 9.20 | H | F | Cl | S | -$CH_3$ | |
| 9.21 | H | F | Cl | S | -$C_2H_5$ | |
| 9.22 | H | F | Cl | S | -$C_3H_7$ | |
| 9.23 | H | F | Cl | S | 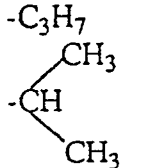 | |
| 9.24 | H | F | Cl | S | $CH_2$-CH=$CH_2$ | |
| 9.25 | H | F | Cl | S | $CH_2$-CHCl=$CH_2$ | |
| 9.26 | H | F | Cl | S | -$CH_2$-CH=CHCl | |

Tabelle 9 (Fortsetzung)

| | R | $R_1$ | $R_2$ | X | $R_4$ | |
|---|---|---|---|---|---|---|
| 9.27 | $CH_3$ | F | Cl | O | $-CH_3$ | |
| 9.28 | $CH_3$ | F | Cl | O | $-C_2H_5$ | |
| 9.29 | $CH_3$ | F | Cl | O | $-C_3H_7$ | |
| 9.30 | $CH_3$ | F | Cl | O | 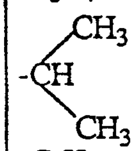 | Smp. 110-112°C |
| 9.31 | $CH_3$ | F | Cl | O | $-C_4H_9$ | |
| 9.32 | $CH_3$ | F | Cl | O | $-CH_2-CH=CH_2$ | |
| 9.33 | $CH_3$ | F | Cl | O | $-CH_2-CHCl=CH_2$ | |
| 9.34 | $CH_3$ | F | Cl | O | $-CH_2-CH=CHCl$ | |
| 9.35 | $CH_3$ | F | Cl | O | $-CH_2-C\equiv CH$ | Smp. 136-138°C |
| 9.36 | $CH_3$ | F | Cl | O | 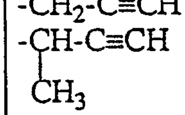 | |
| 9.37 | $CH_3$ | F | Cl | O | $-CH_2-COOCH_3$ | |
| 9.38 | $CH_3$ | F | Cl | O | $-CH_2-COOC_2H_5$ | |
| 9.39 | $CH_3$ | F | Cl | O | $-CH_2-COOC_5H_{11}$ | |
| 9.40 | $CH_3$ | F | Cl | O | $-CH(CH_3)-COOCH_3$ | |
| 9.41 | $CH_3$ | F | Cl | O | $-CH(CH_3)-COOC_5H_{11}$ | |
| 9.42 | $CH_3$ | F | Cl | S | 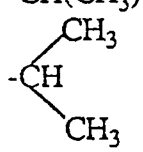 | |
| 9.43 | $CH_3$ | F | Cl | S | $-CH_2-C\equiv CH$ | Smp. 128-130° |
| 9.44 | $CH_3$ | F | Cl | S | $-CH_2-COOCH_3$ | |
| 9.45 | $CH_3$ | F | Cl | S | $-CH_2-COOC_2H_5$ | Oel |
| 9.46 | $CH_3$ | F | Cl | S | $-CH_2-COOC_5H_{11}$ | |
| 9.47 | $CH_3$ | F | Cl | S | $-CH(CH_3)-COOCH_3$ | |
| 9.48 | $CH_3$ | F | Cl | S | $-CH(CH_3)-COOC_2H_5$ | |
| 9.49 | $CH_3$ | F | Cl | S | $-CH(CH_3)-CH_2-OCH_2$ | |
| 9.50 | $CH_3$ | F | Cl | O | $-CH(CH_3)-CH_2-SCH_3$ | |
| 9.51 | $CH_3$ | F | Cl | O | $-CH(CH_3)-CH_2-S-C_2H_5$ | |
| 9.52 | $CH_3$ | F | Cl | O | $-CH(CH_3)-CH_2-S-C_3H_7$ | |
| 9.53 | $CH_3$ | F | Cl | O | $-CH_2-CN$ | |
| 9.54 | $CH_3$ | F | Cl | O | $-CH(CH_3)-CN$ | |
| 9.55 | $CH_3$ | F | Cl | O | $-CH_2-CH_2-Cl$ | |
| 9.56 | $C_3H_7(i)$ | F | Cl | O | 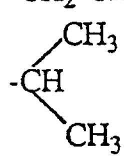 | Smp. 82-84°C |
| 9.57 | $C_3H_7(i)$ | F | Cl | O | $-CH_2-C\equiv CH$ | Smp. 116-118°C |

Tabelle 9 (Fortsetzung)

| | R | $R_1$ | $R_2$ | X | $R_4$ | |
|---|---|---|---|---|---|---|
| 9.58 | $C_3H_7$(i) | F | Cl | O | 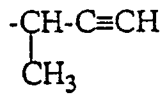 | |
| 9.59 | $C_3H_7$(i) | F | Cl | O | $-CH_2-COOCH_3$ | |
| 9.60 | $C_3H_7$(i) | F | Cl | O | $-CH_2-COOC_2H_5$ | |
| 9.61 | $C_3H_7$(i) | F | Cl | O | $-CH_2-COOC_5H_{11}$ | |
| 9.62 | $C_3H_7$(i) | F | Cl | O | $-CH(CH_3)-COOCH_3$ | |
| 9.63 | $C_3H_7$(i) | F | Cl | O | $-CH(CH_3)-COOC_2H_5$ | |
| 9.64 | $C_3H_7$(i) | F | Cl | O | 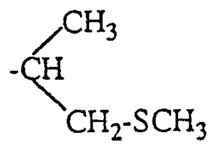 | |
| 9.65 | $C_3H_7$(i) | F | Cl | O | 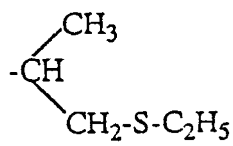 | |
| 9.66 | $C_2H_5$ | F | Cl | O | $-CH_2-C{\equiv}CH$ | Smp. 107-109°C |
| 9.67 | $CH_3$ | F | Cl | O | H | Smp. 174-178°C |
| 9.68 | $C_2H_5$ | F | Cl | O | $-C_3H_7$(i) | Smp. 84-85°C |
| 9.69 | $CF_3$ | F | Cl | O | $-C_3H_7$(i) | Smp. 85-87°C |
| 9.70 | $CH_3$ | F | Cl | O | $-CH_2CH=C(CH_3)_2$ | Smp. 68-70°C |
| 9.71 | $CH_3$ | F | Cl | O | $-CF_3$ | Smp. 79-81°C |
| 9.72 | $CH_3$ | F | Cl | O | 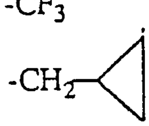 | Smp. 116-118°C |
| 9.73 | $CH_3$ | F | Cl | O | $-CH_2CH_2OCH_3$ | Smp. 88-90°C |
| 9.74 | $CH_3$ | F | Cl | O | $-CH(CH_3)CH=CH_2$ | $n_D^{26}$ 1.5259 |
| 9.75 | $CH_3$ | F | Cl | O | 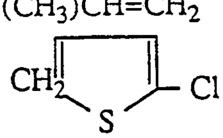 | Smp. 97-99°C |
| 9.76 | $C_2H_5$ | F | Cl | O | $-CH_2COOCH_3$ | $n_D^{23}$ 1.5350 |
| 9.77 | $C_2H_5$ | F | Cl | O | $-CH_2-C{\equiv}CH$ | Smp. 107-109°C |
| 9.78 | $C_2H_5$ | F | Cl | O | $-CH(CH_3)COOCH_3$ | Smp. 74-77°C |
| 9.79 | $C_3H_7$(i) | F | Cl | O | $-CH_2-CH=CH_2$ | Smp. 82-84°C |
| 9.80 | $C_3H_7$(i) | F | Cl | O | $-CH_2CN$ | Smp. 99-102°C |
| 9.81 | $C_3H_7$(i) | F | Cl | O | 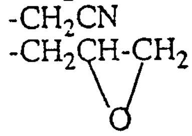 | Smp. 103-104°C |
| 9.82 | $C_3H_7$(i) | F | Cl | O | $-CH_2CH=CH-COOCH_3$ | Smp. 94-97°C |

Tabelle 9 (Fortsetzung)

| | R | $R_1$ | $R_2$ | X | $R_4$ | |
|---|---|---|---|---|---|---|
| 9.83 | $CF_3CF_2$ | F | Cl | O | $-C_3H_7(i)$ | |
| 9.84 | $CF_3CF_2$ | F | Cl | O | $-CH_2C{\equiv}CH$ | |
| 9.85 | $CF_3CF_2CF_2$ | F | Cl | O | $-C_3H_7(i)$ | |
| 9.86 | $CF_3CF_2CF_2$ | F | Cl | O | $-CH_2C{\equiv}CH$ | |
| 9.87 | $CF_3$ | F | Cl | O | $-CH_2C{\equiv}CH$ | Smp. 103-105°C |
| 9.88 | $CF_3$ | F | Cl | O | $CH_2-C{\equiv}CH$ | |
| 9.89 | $CF_3$ | F | Cl | O | 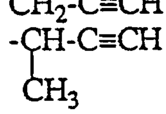 | |
| 9.90 | $CF_3$ | F | Cl | O | $-CH_2-COOC_5H_{11}$ | |
| 9.91 | $CF_3$ | F | Cl | O | $-CH_2-COOCH_3$ | |
| 9.92 | $CF_3$ | F | Cl | O | $-CH_2-COOC_2H_5$ | |
| 9.93 | $CF_3$ | F | Cl | O | $-CH_2-COOCH_3$ | |
| 9.94 | $CF_3$ | F | Cl | O | $-CH_3$ | |
| 9.95 | $CF_3$ | F | Cl | O | $-C_2H_5$ | |
| 9.96 | $CF_3$ | F | Cl | O | $-C_3H_7$ | |
| 9.97 | $CF_3$ | F | Cl | O | 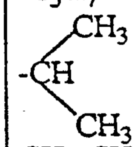 | |
| 9.98 | $CF_3$ | F | Cl | O | $CH_2-CH=CH_2$ | |
| 9.99 | $CF_3$ | F | Cl | O | $-CH_2-CHCl=CH_2$ | |
| 9.100 | $CF_3$ | F | Cl | O | $-CH_2-CH=CHCl$ | |
| 9.101 | $CF_3$ | F | Cl | S | $-CH_2-C{\equiv}CH$ | |
| 9.102 | $CF_3$ | F | Cl | S | 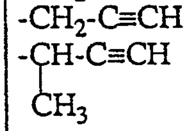 | |
| 9.103 | $CF_3$ | F | Cl | S | $-CH_2-COOC_5H_{11}$ | |
| 9.104 | $CF_3$ | F | Cl | S | $-CH_2-COOCH_3$ | |
| 9.105 | $CF_3$ | F | Cl | S | $-CH_2-COOC_2H_5$ | |
| 9.106 | $CF_3$ | F | Cl | S | $-CH_2(CH_3)-COOCH_3$ | |
| 9.107 | $CF_3$ | F | Cl | S | $-CH_3$ | |
| 9.108 | $CF_3$ | F | Cl | S | $-C_2H_5$ | |
| 9.109 | $CF_3$ | F | Cl | S | $-C_3H_7$ | |
| 9.110 | $CF_3$ | F | Cl | S | 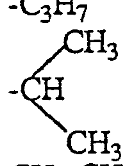 | |
| 9.111 | $CF_3$ | F | Cl | S | $CH_2-CH=CH_2$ | |
| 9.112 | $CF_3$ | F | Cl | S | $CH_2-CHCl=CH_2$ | |
| 9.113 | $CF_3$ | F | Cl | S | $-CH_2-CH=CHCl$ | |
| 9.114 | $C_3H_7i$ | F | Cl | S | $-CH_2-CH=CHCl$ | |

Tabelle 9 (Fortsetzung)

| | R | $R_1$ | $R_2$ | X | $R_4$ | |
|---|---|---|---|---|---|---|
| 9.115 | H | F | Br | O | $CH_2$-C≡CH | |
| 9.116 | H | F | Br | O | 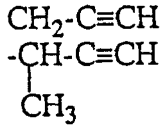 | |
| 9.117 | H | F | Br | O | $-CH_2-COOC_5H_{11}$ | |
| 9.118 | H | F | Br | O | $-CH_2-COOCH_3$ | |
| 9.119 | H | F | Br | O | $-CH_2-COOC_2H_5$ | |
| 9.120 | H | F | Br | O | $-CH_2-COOCH_3$ | |
| 9.121 | H | F | Br | O | $-CH_3$ | |
| 9.122 | H | F | Br | O | $-C_2H_5$ | |
| 9.123 | H | F | Br | O | $-C_3H_7$ | |
| 9.124 | H | F | Br | O | 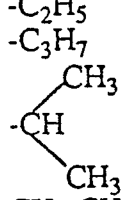 | |
| 9.125 | H | F | Br | O | $CH_2-CH=CH_2$ | |
| 9.126 | H | F | Br | O | $-CH_2-CHCl=CH_2$ | |
| 9.127 | H | F | Br | O | $-CH_2-CH=CHCl$ | |
| 9.128 | H | F | Br | S | $-CH_2$-C≡CH | |
| 9.129 | H | F | Br | S | 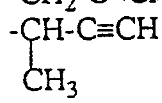 | |
| 9.130 | H | F | Br | S | $-CH_2-COOC_5H_{11}$ | |
| 9.131 | H | F | Br | S | $-CH_2-COOCH_3$ | |
| 9.132 | H | F | Br | S | $-CH_2-COOC_2H_5$ | |
| 9.133 | H | F | Br | S | $-CH_2(CH_3)-COOCH_3$ | |
| 9.134 | H | F | Br | S | $-CH_3$ | |
| 9.135 | H | F | Br | S | $-C_2H_5$ | |
| 9.136 | H | F | Br | S | $-C_3H_7$ | |
| 9.137 | H | F | Br | S | 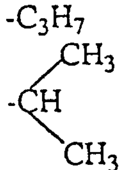 | |
| 9.138 | H | F | Br | S | $CH_2-CH=CH_2$ | |
| 9.139 | H | F | Br | S | $CH_2-CHCl=CH_2$ | |
| 9.140 | H | F | Br | S | $-CH_2-CH=CHCl$ | |

Tabelle 9 (Fortsetzung)

| | R | $R_1$ | $R_2$ | X | $R_4$ | |
|---|---|---|---|---|---|---|
| 9.141 | $CH_3$ | F | Br | O | $-CH_3$ | |
| 9.142 | $CH_3$ | F | Br | O | $-C_2H_5$ | |
| 9.143 | $CH_3$ | F | Br | O | $-C_3H_7$ | |
| 9.144 | $CH_3$ | F | Br | O | 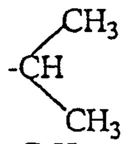 | |
| 9.145 | $CH_3$ | F | Br | O | $-C_4H_9$ | |
| 9.146 | $CH_3$ | F | Br | O | $-CH_2-CH=CH_2$ | |
| 9.147 | $CH_3$ | F | Br | O | $-CH_2-CHCl=CH_2$ | |
| 9.148 | $CH_3$ | F | Br | O | $-CH_2-CH=CHCl$ | |
| 9.149 | $CH_3$ | F | Br | O | $-CH_2-C{\equiv}CH$ | |
| 9.150 | $CH_3$ | F | Br | O | 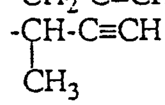 | |
| 9.151 | $CH_3$ | F | Br | O | $-CH_2-COOCH_3$ | |
| 9.152 | $CH_3$ | F | Br | O | $-CH_2-COOC_2H_5$ | |
| 9.153 | $CH_3$ | F | Br | O | $-CH_2-COOC_5H_{11}$ | |
| 9.154 | $CH_3$ | F | Br | O | $-CH(CH_3)-COOCH_3$ | |
| 9.155 | $CH_3$ | F | Br | O | $-CH(CH_3)-COOC_5H_{11}$ | |
| 9.156 | $CH_3$ | F | Br | S | 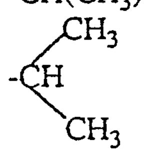 | |
| 9.157 | $CH_3$ | F | Br | S | $-CH_2-C{\equiv}CH$ | |
| 9.158 | $CH_3$ | F | Br | S | $-CH_2-COOCH_3$ | |
| 9.159 | $CH_3$ | F | Br | S | $-CH_2-COOC_2H_5$ | |
| 9.160 | $CH_3$ | F | Br | S | $-CH_2-COOC_5H_{11}$ | |
| 9.161 | $CH_3$ | F | Br | S | $-CH(CH_3)-COOCH_3$ | |
| 9.162 | $CH_3$ | F | Br | S | $-CH(CH_3)-COOC_2H_5$ | |
| 9.163 | $CH_3$ | F | Br | S | $-CH(CH_3)-CH_2-OCH_2$ | |
| 9.164 | $CH_3$ | F | Br | O | $-CH(CH_3)-CH_2-SCH_3$ | |
| 9.165 | $CH_3$ | F | Br | O | $-CH(CH_3)-CH_2-S-C_2H_5$ | |
| 9.166 | $CH_3$ | F | Br | O | $-CH(CH_3)-CH_2-S-C_3H_7$ | |
| 9.167 | $CH_3$ | F | Br | O | $-CH_2-CN$ | |
| 9.168 | $CH_3$ | F | Br | O | $-CH(CH_3)-CN$ | |
| 9.169 | $CH_3$ | F | Br | O | $-CH_2-CH_2-Cl$ | |
| 9.170 | $C_3H_7(i)$ | F | Br | O | 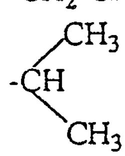 | |
| 9.171 | $C_3H_7(i)$ | F | Br | O | $-CH_2-C{\equiv}CH$ | |

Tabelle 9 (Fortsetzung)

| | R | $R_1$ | $R_2$ | X | $R_4$ | |
|---|---|---|---|---|---|---|
| 9.172 | $C_3H_7$(i) | F | Br | O | 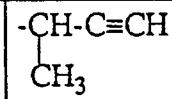 | |
| 9.173 | $C_3H_7$(i) | F | Br | O | $-CH_2-COOCH_3$ | |
| 9.174 | $C_3H_7$(i) | F | Br | O | $-CH_2-COOC_2H_5$ | |
| 9.175 | $C_3H_7$(i) | F | Br | O | $-CH_2-COOC_5H_{11}$ | |
| 9.176 | $C_3H_7$(i) | F | Br | O | $-CH(CH_3)-COOCH_3$ | |
| 9.177 | $C_3H_7$(i) | F | Br | O | $-CH(CH_3)-COOC_2H_5$ | |
| 9.178 | $C_3H_7$(i) | F | Br | O | 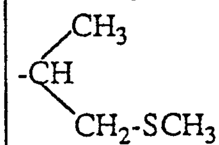 | |
| 9.179 | $C_3H_7$(i) | F | Br | O | 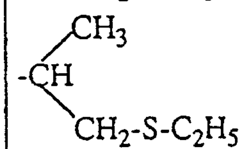 | |
| 9.180 | $C_2H_5$ | F | Br | O | $-CH_2-C\equiv CH$ | |
| 9.181 | $CH_3$ | F | Br | O | H | |
| 9.182 | $C_2H_5$ | F | Br | O | $-C_3H_7$(i) | |
| 9.183 | $CF_3$ | F | Br | O | $-C_3H_7$(i) | |
| 9.184 | $CH_3$ | F | Br | O | $-CH_2CH=C(CH_3)_2$ | |
| 9.185 | $CH_3$ | F | Br | O | $-CF_3$ | |
| 9.186 | $CH_3$ | F | Br | O | 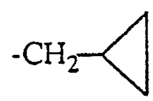 | |
| 9.187 | $CH_3$ | F | Br | O | $-CH_2CH_2OCH_3$ | |
| 9.188 | $CH_3$ | F | Br | O | $-CH(CH_3)CH=CH_2$ | |
| 9.189 | $CH_3$ | F | Br | O | 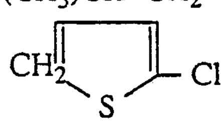 | |
| 9.190 | $C_2H_5$ | F | Br | O | $-CH_2COOCH_3$ | |
| 9.191 | $C_2H_5$ | F | Br | O | $-CH_2-C\equiv CH$ | |
| 9.192 | $C_2H_5$ | F | Br | O | $-CH(CH_3)COOCH_3$ | |
| 9.193 | $C_3H_7$(i) | F | Br | O | $-CH_2-CH=CH_2$ | |
| 9.194 | $C_3H_7$(i) | F | Br | O | $-CH_2CN$ | |
| 9.195 | $C_3H_7$(i) | F | Br | O | 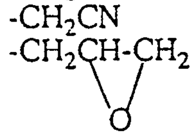 | |
| 9.196 | $C_3H_7$(i) | F | Br | O | $-CH_2CH=CH-COOCH_3$ | |

Tabelle 9 (Fortsetzung)

| | R | $R_1$ | $R_2$ | X | $R_4$ | |
|---|---|---|---|---|---|---|
| 9.197 | $CF_3CF_2$ | F | Br | O | $-C_3H_7(i)$ | |
| 9.198 | $CF_3CF_2$ | F | Br | O | $-CH_2C\equiv CH$ | |
| 9.199 | $CF_3CF_2CF_2$ | F | Br | O | $-C_3H_7(i)$ | |
| 9.200 | $CF_3CF_2CF_2$ | F | Br | O | $-CH_2C\equiv CH$ | |
| 9.201 | $CF_3$ | F | Br | O | $-CH_2C\equiv CH$ | |
| 9.202 | $CF_3$ | F | Br | O | $CH_2-C\equiv CH$ | |
| 9.203 | $CF_3$ | F | Br | O | 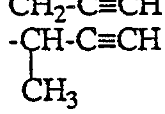 | |
| 9.204 | $CF_3$ | F | Br | O | $-CH_2-COOC_5H_{11}$ | |
| 9.205 | $CF_3$ | F | Br | O | $-CH_2-COOCH_3$ | |
| 9.206 | $CF_3$ | F | Br | O | $-CH_2-COOC_2H_5$ | |
| 9.207 | $CF_3$ | F | Br | O | $-CH_2-COOCH_3$ | |
| 9.208 | $CF_3$ | F | Br | O | $-CH_3$ | |
| 9.209 | $CF_3$ | F | Br | O | $-C_2H_5$ | |
| 9.210 | $CF_3$ | F | Br | O | $-C_3H_7$ | |
| 9.211 | $CF_3$ | F | Br | O | 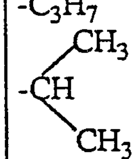 | |
| 9.212 | $CF_3$ | F | Br | O | $CH_2-CH=CH_2$ | |
| 9.213 | $CF_3$ | F | Br | O | $-CH_2-CHCl=CH_2$ | |
| 9.214 | $CF_3$ | F | Br | O | $-CH_2-CH=CHCl$ | |
| 9.215 | $CF_3$ | F | Br | S | $-CH_2-C\equiv CH$ | |
| 9.216 | $CF_3$ | F | Br | S | 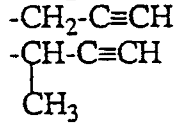 | |
| 9.217 | $CF_3$ | F | Br | S | $-CH_2-COOC_5H_{11}$ | |
| 9.218 | $CF_3$ | F | Br | S | $-CH_2-COOCH_3$ | |
| 9.219 | $CF_3$ | F | Br | S | $-CH_2-COOC_2H_5$ | |
| 9.220 | $CF_3$ | F | Br | S | $-CH_2(CH_3)-COOCH_3$ | |
| 9.221 | $CF_3$ | F | Br | S | $-CH_3$ | |
| 9.222 | $CF_3$ | F | Br | S | $-C_2H_5$ | |
| 9.223 | $CF_3$ | F | Br | S | $-C_3H_7$ | |
| 9.224 | $CF_3$ | F | Br | S | 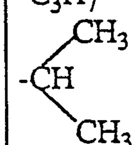 | |
| 9.225 | $CF_3$ | F | Br | S | $CH_2-CH=CH_2$ | |
| 9.226 | $CF_3$ | F | Br | S | $CH_2-CHCl=CH_2$ | |
| 9.227 | $CF_3$ | F | Br | S | $-CH_2-CH=CHCl$ | |
| 9.228 | $C_3H_7i$ | F | Br | S | $-CH_2-CH=CHCl$ | |
| 9.229 | $C_4H_9t$ | F | Cl | S | $-CH_2COOCH_3$ | $n_D^{22}$: 1.5432 |
| 9.230 | $C_4H_9t$ | F | Cl | O | $-CH_2C\equiv CH$ | Smp.: 169-171° |
| 9.231 | 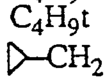 | F | Cl | O | $-CH_2C\equiv CH$ | Smp.: 121-123° |
| 9.232 | $C_3H_7i$ | F | Cl | S | $-CH_2COOC_2H_5$ | Smp.: 70-72° |

Verbindungen der Formel

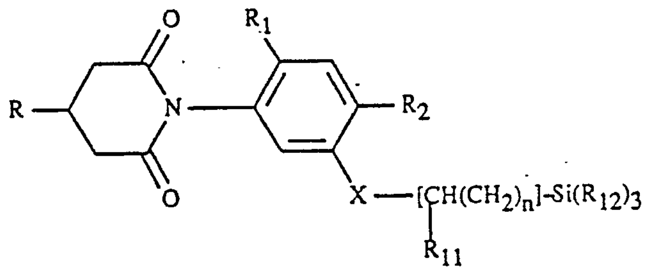

(Ii)

Tabelle 10

| | R | $R_1$ | $R_2$ | X | $R_{11}$ | n | $R_{12}$ |
|---|---|---|---|---|---|---|---|
| 10.01 | $CH_3$ | F | Cl | O | H | 0 | $CH_3$ |
| 10.02 | $CH_3$ | F | Cl | O | H | 1 | $CH_3$ |
| 10.03 | $CH_3$ | F | Cl | O | $CH_3$ | 0 | $CH_3$ |
| 10.04 | $C_2H_5$ | F | Br | O | $CH_3$ | 0 | $CH_3$ |
| 10.05 | $C_2H_5$ | F | Br | O | $CH_3$ | 1 | $CH_3$ |
| 10.06 | $C_2H_5$ | F | Cl | O | H | 1 | $C_2H_5$ |
| 10.07 | $CH_3$ | F | Cl | S | H | 0 | $CH_3$ |
| 10.08 | $CH_3$ | F | Cl | S | H | 1 | $CH_3$ |
| 10.09 | $CH_3$ | F | Cl | S | $CH_3$ | 0 | $CH_3$ |
| 10.10 | $C_3H_7$(i) | F | Br | S | $CH_3$ | 0 | $CH_3$ |
| 10.11 | $C_3H_7$(i) | F | Br | S | $CH_3$ | 1 | $CH_3$ |
| 10.12 | $CH_3$ | F | Cl | S | $C_2H_5$ | 0 | $CH_3$ |
| 10.13 | $CH_3$ | F | Br | S | $C_2H_5$ | 1 | $CH_3$ |
| 10.14 | $CF_3$ | F | Cl | O | $CH_3$ | 1 | $CH_3$ |
| 10.15 | $CF_3$ | F | Cl | S | $CH_3$ | 1 | $CH_3$ |
| 10.16 | $CF_3$ | F | Cl | S | H | 0 | $CH_3$ |
| 10.17 | $CF_3$ | F | Cl | O | H | 0 | $CH_3$ |

Verbindungen der Formel

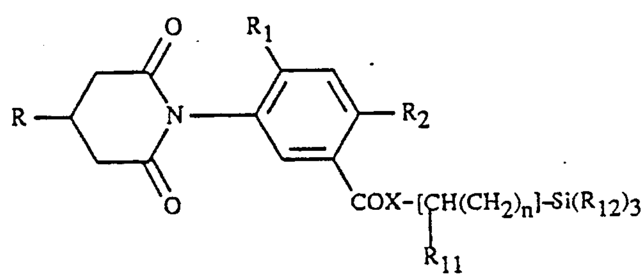

(Ij)

Tabelle 11

| | R | $R_1$ | $R_2$ | X | $R_{11}$ | n | $R_{12}$ |
|---|---|---|---|---|---|---|---|
| 11.01 | $CH_3$ | F | Cl | O | H | 0 | $CH_3$ |
| 11.02 | $CH_3$ | F | Cl | O | H | 1 | $CH_3$ |
| 11.03 | $CH_3$ | F | Cl | O | $CH_3$ | 1 | $CH_3$ |
| 11.04 | $CH_3$ | F | Cl | O | $CH_3$ | 1 | $C_2H_5$ |
| 11.05 | $CH_3$ | F | Cl | S | H | 0 | $CH_3$ |
| 11.06 | $CH_3$ | F | Cl | S | H | 1 | $CH_3$ |
| 11.07 | $CH_3$ | F | Cl | S | $CH_3$ | 1 | $CH_3$ |
| 11.08 | $C_2H_5$ | F | Br | O | H | 0 | $CH_3$ |
| 11.09 | $C_2H_5$ | F | Br | O | H | 1 | $CH_3$ |
| 11.10 | $C_2H_5$ | F | Br | O | $CH_3$ | 1 | $CH_3$ |
| 11.11 | $C_3H_7$(i) | F | Br | S | H | 0 | $CH_3$ |
| 11.12 | $C_3H_7$(i) | F | Br | S | H | 1 | $CH_3$ |
| 11.13 | $C_3H_7$(i) | F | Br | S | $CH_3$ | 1 | $CH_3$ |
| 11.14 | $CH_3$ | F | Cl | O | $C_2H_5$ | 1 | $CH_3$ |
| 11.15 | $CH_3$ | F | Cl | S | $C_2H_5$ | 1 | $CH_3$ |
| 11.16 | $CH_3$ | F | Br | O | $C_2H_5$ | 1 | $CH_3$ |
| 11.17 | $CF_3$ | F | Cl | O | $CH_3$ | 1 | $CH_3$ |
| 11.18 | $CF_3$ | F | Cl | S | $CH_3$ | 1 | $CH_3$ |
| 11.19 | $CF_3$ | F | Cl | S | H | 0 | $CH_3$ |
| 11.20 | $CF_3$ | F | Cl | O | H | 0 | $CH_3$ |

B. Biologische Beispiele

Beispiel B 1: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %-igen Emulsionskonzentrat behandelt. Es werden unterschiedliche Aufwandmengen Wirksubstanz/Hektar getestet. Die Saatschalen werden im Gewächshaus bei 22-25 °C und 50-70 % relativer Luftfeuchtigkeit gehalten und der Versuch nach 3 Wochen ausgewertet.

Die Herbizidwirkung wird dabei in einem neunstufigen (1 = vollständige Schädigung der Versuchspflanze, 9 = keine Herbizidwirkung an der Versuchspflanze) Boniturschema im Vergleich zur unbehandelten Kontrollgruppe ausgewertet.

Boniturnoten von 1 bis 4 (insbesondere von 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Test zeigt zum Beispiel die geprüften Verbindungen der Tabellen 1-11 bei Aufwandmengen von 125 bis 500 [g]AS/[ha] allgemein gute Herbizidwirkung bei Digitaria sang., Cyperus esculentus, Abutilon, Sida spinosa, Amaranthus retroflexus, Chenopodium sp., Solanum nigrum, Sinapis, Viola tricolor und Veronica sp. bei gleichzeitig guter Kulturpflanzenverträglichkeit in Soja, Baumwolle und Sonnenblumenkulturen.

Einzelresultate sind in der Tabelle 12 zusammengefasst.

TABELLE 12

| Verbindung Nr. | 8.38 | | | 8.73 | | | 9.35 | | | 9.57 | | | 9.77 | | |
|---|---|---|---|---|---|---|---|---|---|---|---|---|---|---|---|
| Aufwandmenge g/ha | 500 | 250 | 125 | 500 | 250 | 125 | 500 | 250 | 125 | 500 | 250 | 125 | 500 | 250 | 125 |
| Weizen | 6 | 7 | 8 | 6 | 6 | 9 | 5 | 6 | 8 | 5 | 6 | 7 | 5 | 6 | 8 |
| Hirse | 7 | 9 | 9 | 8 | 9 | 9 | 5 | 6 | 9 | 7 | 8 | 9 | 6 | 7 | 9 |
| Soya | 9 | 9 | 9 | 6 | 7 | 8 | 6 | 7 | 9 | 7 | 9 | 9 | 5 | 7 | 9 |
| Baumwolle | 7 | 9 | 9 | 8 | 9 | 9 | 4 | 6 | 7 | 8 | 9 | 9 | 7 | 7 | 8 |
| Sonnenblume | 8 | 9 | 9 | 8 | 9 | 9 | 6 | 6 | 7 | 7 | 7 | 9 | 5 | 7 | 8 |
| Avena fatua | 1 | 3 | 6 | 3 | 4 | 6 | 1 | 1 | 1 | 2 | 3 | 5 | 1 | 4 | 5 |
| Lolium perenne | 3 | 4 | 4 | 4 | 4 | 5 | 1 | 2 | 3 | 1 | 4 | 5 | 1 | 2 | 5 |
| Digitaria sang. | 1 | 1 | 4 | 1 | 1 | 1 | 1 | 1 | 1 | 1 | 1 | 3 | 1 | 1 | 3 |
| Abutilon | 1 | 1 | 4 | 1 | 1 | 1 | 1 | 1 | 1 | 1 | 1 | 1 | 1 | 1 | 1 |
| Sida spinosa | 1 | 1 | 1 | 1 | 1 | 1 | 1 | 1 | 1 | 1 | 1 | 1 | 1 | 1 | 1 |
| Amaranthus | 1 | 1 | 1 | 1 | 1 | 1 | 1 | 1 | 1 | 1 | 1 | 3 | 1 | 1 | 1 |
| Chenopodium album | 1 | 1 | 1 | 1 | 1 | 1 | 1 | 1 | 1 | 1 | 1 | 1 | 1 | 1 | 1 |
| Solanum nigrum | 3 | 3 | 4 | 1 | 1 | 4 | 1 | 1 | 4 | 1 | 1 | 1 | 1 | 1 | 1 |
| Sinapis album | 1 | 3 | 4 | 3 | 3 | 5 | 1 | 1 | 2 | 1 | 2 | 5 | 1 | 3 | 4 |
| Chrysanthemum | 1 | 2 | 4 | 1 | 3 | 3 | 1 | 2 | 5 | 1 | 1 | 3 | 1 | 2 | 6 |
| Viola tricolor | 1 | 1 | 1 | 1 | 1 | 1 | 1 | 1 | 1 | 1 | 1 | 1 | 1 | 1 | 2 |
| Veronica sp. | 1 | 1 | 1 | 1 | 1 | 1 | 1 | 1 | 1 | 1 | 1 | 3 | 1 | 2 | 4 |

Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 125 bis 500 g Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach der obigen Notenskala ausgewertet. Auch in diesem Versuch zeigen die geprüften Verbindungen der Tabellen 1 bis 11 allgemein starke bis sehr starke Herbizidwirkung.

Einzelresultate sind in der Tabelle 13 zusammengefasst.

Tabelle 13

| Verbindung Nr. | 8.73 | | | 9.35 | | | 9.57 | | | 9.77 | | |
|---|---|---|---|---|---|---|---|---|---|---|---|---|
| Aufwandmenge g/ha | 500 | 250 | 125 | 500 | 250 | 125 | 500 | 250 | 125 | 500 | 250 | 125 |
| Hirse | 6 | 6 | 6 | 5 | 7 | 8 | 6 | 7 | 8 | 6 | 6 | 7 |
| Avena fatua | 4 | 5 | 5 | 2 | 2 | 4 | 2 | 4 | 4 | 1 | 2 | 3 |
| Lolium perenne | 2 | 3 | 4 | 1 | 1 | 2 | 1 | 3 | 4 | 1 | 1 | 2 |
| Cyperus asalentus | 3 | 4 | 4 | 1 | 2 | 2 | 1 | 2 | 3 | 1 | 2 | 3 |
| Abutilon | 1 | 1 | 1 | 1 | 1 | 2 | 1 | 1 | 2 | 1 | 1 | 1 |
| Sida spinosa | 1 | 1 | 1 | 2 | 2 | 3 | 1 | 2 | 2 | 1 | 2 | 2 |
| Xanthium sp. | 1 | 1 | 4 | 3 | 3 | 3 | 2 | 2 | 2 | 2 | 3 | 4 |
| Amaranthus ret. | 2 | 3 | 4 | 1 | 1 | 3 | 2 | 3 | 4 | 1 | 1 | 2 |
| Chenopodium sp. | 1 | 1 | 1 | 2 | 2 | 3 | 1 | 2 | 3 | 1 | 1 | 2 |
| Solanum nigrum | 1 | 1 | 2 | 1 | 1 | 1 | 1 | 1 | 1 | 1 | 1 | 1 |
| Ipomoea purp. | 1 | 1 | 1 | 1 | 1 | 2 | 1 | 1 | 1 | 1 | 1 | 1 |
| Sinapis album | 1 | 2 | 3 | 1 | 1 | 2 | 1 | 1 | 1 | 1 | 1 | 1 |
| Chrysanthemum leuc. | 1 | 2 | 2 | 3 | 3 | 4 | 1 | 2 | 2 | 1 | 1 | 2 |
| Galium aparine | 2 | 2 | 2 | 2 | 2 | 2 | 2 | 2 | 3 | 2 | 2 | 2 |
| Viola tricolor | 1 | 1 | 1 | 1 | 1 | 1 | 1 | 1 | 1 | 1 | 1 | 1 |
| Veronica sp. | 1 | 1 | 1 | 2 | 2 | 2 | 1 | 3 | 3 | 1 | 1 | 1 |

Beispiel B3: Herbizidwirkung vor dem Auflaufen der Pflanzen

Kunststofföpfe werden mit expandiertem Vermiculit (Dichte: 0,135 g/cm$^3$, Wasserabsorptionsvermögen: 0,565 1/1) gefüllt. Nach dem Sättigen des nicht adsorptiven Vermiculits mit einer wässrigen Wirkstoffemulsion in deinonisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deionisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet.

Die geprüften Verbindungen der Tabellen 1-11 zeigen in diesem generell Versuch gute Herbizidwirkung.

Beispiel B4: Herbizidwirkung für Wasserreis (paddy)

Die Wasserunkräuter Echinochloa crus galli und Monocharia vag. werden in Plastikbechern (60 cm$^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die daher verwendete Dosis entspricht einer Wirkstoffmenge von 60 bis 500 g AS pro Hektar (Spritzbrühmenge = 550 l/ha. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Die geprüften Verbindungen der Tabellen 1 bis 11 schädigen dabei die Unkräuter, nicht aber den Reis.

Einzelresultate sind in der Tabelle 14 zusammengefasst.

Tabelle 14

| Verbindung Nr. | 9.35 | | | | 9.57 | | | | 9.77 | | | |
|---|---|---|---|---|---|---|---|---|---|---|---|---|
| Aufwandmenge g/ha | 500 | 250 | 125 | 60 | 500 | 250 | 125 | 60 | 500 | 250 | 125 | 60 |
| Reis | 4 | 5 | 6 | 7 | 6 | 7 | 8 | 9 | 5 | 7 | 7 | 8 |
| Echinochloa crus galli | 3 | 4 | 4 | 4 | 1 | 2 | 3 | 3 | 2 | 2 | 3 | 4 |
| Scirpus sp. | 4 | 4 | 4 | 5 | 1 | 2 | 4 | 4 | 2 | 2 | 2 | 4 |
| Monucharia vag. | 1 | 1 | 1 | 1 | 1 | 1 | 1 | 1 | 1 | 1 | 1 | 1 |
| Sagittaria sp. | 1 | 1 | 3 | 4 | 1 | 1 | 1 | 1 | 1 | 1 | 1 | 1 |

Beispiel B5: Wuchshemmung bei tropischen Bodenbedecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit Wirkstoffen der Tabellen 1 bis 11 in Aufwandmengen von 50-3000 g/ha behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel B6: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die geprüften erfindungsgemässen Wirkstoffe der Tabellen 1 bis 11 eine merklicher Erhöhung der Anzahl und des Gewichts der Schoten im Haupttrieb.

Beispiel B7: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes besprüht. Die Wirkstoffmenge beträgt bis zu 100 g Aktivsubstanz pro Hektar. 21 Tage nach Applikation wird das Wachstum des Getreides beurteilt. Die mit erfindungsgemässen Wirkstoffen behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel B8: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerata und Cynodon dactylon im Gewächshaus angesät und nach Bedarf bewässert. Die aufgeleufenen Gräser werden wöchentlich bis auf 4 cm Höhe zurückgeschnitten und

ca. 50 Tage nach der Aussaat und einen Tag nach dem letzten Schnitt mit der wässrigen Spritzbrühe eines Wirkstoffes be sprüht. Die Wirkstoffmenge beträgt umgerechnet bis zu 500 g Aktivsubstanz pro Hektar. 21 Tage nach Applikation wird das Wachstum der Gräser beurteilt.

Geprüfte Verbindungen der Tabellen 1 bis 11 bewirken eine Reduzierung des Neuzuwachses von um 10-30 % im Vergleich zur unbehandelten Kontrolle.

Beispiel B9: Desiccations- und Defoliationswirkung

In einem Gewächshaus wurden Baumwollpflanzen der Sorte Deltapine in Tontöpfen angezogen. Nach abgeschlossener Kapselbildung wurden sie mit wässerigen Zubereitungen eines Wirkstoffes in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Desiccation (% Austrocknung der an der Pflanze verbleibenden Blätter).

In diesem Versuch verblieben auf den mit Verbindungen der Tabellen 1-11 bei Aufwandmengen von 0,6 und 1,2 kg/ha gespritzten Pflanzen nach 7 Tagen bloss noch wenige vertrocknete Blätter auf den Pflanzen (> 80 % Blattfall und Austrocknung).

Formulierungsbeispiele

Beispiel F1: Formulierungsbeispiele für Wirkstoffe der Formel I′ (% = Gewichtsprozent)

| a) Emulsionskonzentrate | a) | b) | c) |
|---|---|---|---|
| Wirkstoff gemäss Tabellen 1-11 | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenol-polyäthylenglykoläther (30 Mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) |
|---|---|---|---|
| Wirkstoff gemäss Tabellen 1-11 | 80 % | 10 % | 5 % |
| Äthylenglykol-monomethyläther | 20 % | - | - |
| Polyäthylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | a) | b) |
|---|---|---|
| Wirkstoff gemäss Tabellen 1-11 | 5 % | 10 % |
| Kaolin | 94 % | - |
| Hochdisperse Kieselsäure | 1 % | |
| Attapulgit | - | 90 % |

Eine Wirkstofflösung wird auf den Träger aufgesprüht und das Lösungsmittel anschliessend im Vakuum abgedampft.

| d) Stäubemittel | a) | b) |
|---|---|---|
| Wirkstoff gemäss Tabellen 1-11 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertiges Stäubemittel.

| e) Spritzpulver | a) | b) |
|---|---|---|
| Wirkstoff gemäss Tabellen 1-11 | 20 % | 60 % |
| Na-Ligninsulfonat | 5 % | 5 % |
| Na-Laurylsulfat | - | 6 % |
| Octylphenolpolyäthylenglykoläther (7-8 Mol Ae) | - | 2 % |
| Hochdisperse Kieselsäure | 5 % | 27 % |
| Kaolin | 70 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

| f) Extruder Granulat | |
|---|---|
| Wirkstoff gemäss Tabellen 1-11 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| g) Umhüllungs-Granulat | |
|---|---|
| Wirkstoff gemäss Tabellen 1-11 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| h) Suspensions-Konzentrat | |
|---|---|
| Wirkstoff gemäss Tabellen 1-11 | 40 % |
| Äthylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %-ige wässrige Formaldehyd-Lösung | 0,2 % |
| Silkonöl in Form einer 75 %-igen wässrigen Emulsion | 0,8 % |
| Wasser | ad 100 % |

Der Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Ansprüche

1. Neue 1-Phenyl-piperidin-2,6-dione der Formel

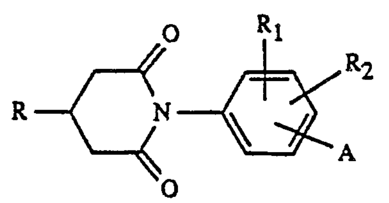 (I),

worin

R Wasserstoff; $C_1$-$C_6$-Alkyl; $C_3$-$C_7$-Cycloalkyl; $C_1$-$C_4$-Alkoxy-$C_1$-$C_4$-alkyl; $C_1$-$C_4$-Alkylthio-$C_1$-$C_4$-alkyl; Halogen-$C_1$-$C_7$-alkyl; $C_3$-$C_7$-Alkenyl; $C_3$-$C_7$-Alkinyl;

$R_1$ Wasserstoff; Halogen;

$R_2$ Wasserstoff; Cyano; Nitro; Halogen;

$R_3$ Halogen; $X'$-$R_5$; Amino; $C_1$-$C_4$-Alkylamino; Di-$C_1$-$C_4$-alkylamino; $C_2$-$C_4$-Halogenalkylamino; Di-$C_2$-$C_4$-halogenalkylamino; $C_1$-$C_4$-Hydroxyalkylamino; Di-$C_1$-$C_4$-hydroxyalkylamino; $C_3$-$C_4$-Alkenylamino; Diallylamino; -N-Pyrrolidino; -N-Piperidino; -N-Morpholino; -N-Thiomorpholino; -N-Piperidazino;

A Wasserstoff; Cyano; Nitro; $COR_3$; $XR_4$;

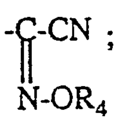 ; $-COR_8$; 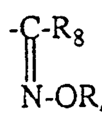 ; 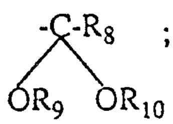 ;

$-\underset{\displaystyle O}{\overset{\|}{C}}$-X[$CHR_{11}(CH_2)_n$]-Si$(R_{12})_3$

und -X-[$CHR_{11}$-$(CH_2)_n$]-Si$(R_{12})_3$;

$R_4$ Wasserstoff, $C_1$-$C_{10}$-Alkyl; $C_1$-$C_4$-Alkoxy-$C_1$-$C_4$-alkyl; $C_1$-$C_4$-Alkylthio-$C_1$-$C_4$-alkyl; Di-$C_1$-$C_4$-alkylamino-$C_1$-$C_4$-alkyl; Halogen-$C_1$-$C_8$-alkyl; $C_2$-$C_8$-Alkenyl; Halogen-$C_2$-$C_8$-alkenyl; $C_3$-$C_8$-Alkinyl; $C_3$-$C_7$-Cycloalkyl; Halogen-$C_3$-$C_7$-cycloalkyl; $C_1$-$C_8$-Alkylcarbonyl; Allylcarbonyl; $C_3$-$C_7$-Cycloalkylcarbonyl; Benzoyl, das unsubstituiert oder am Phenylring gleich oder verschieden bis zu dreifach durch Halogen, $C_1$-$C_4$-Alkyl, Halogen-$C_1$-$C_4$-alkyl, Halogen-$C_1$-$C_4$-alkoxy oder $C_1$-$C_4$-Alkoxy, substituiert ist; Furanoyl; Thiophenoyl; oder $C_1$-$C_4$-Alkyl substituiert durch Phenyl, Halogenphenyl, $C_1$-$C_4$-Alkylphenyl, $C_1$-$C_4$-Alkoxyphenyl, Halogen-$C_1$-$C_4$-alkylphenyl, Halogen-$C_1$-$C_4$-alkoxyphenyl, $C_1$-$C_6$-Alkoxycarbonyl, $C_1$-$C_4$-Alkoxy-$C_1$-$C_8$-alkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl, $C_3$-$C_8$-Alkinylthiocarbonyl, Carbamoyl, Mono-$C_1$-$C_4$-alkylaminocarbonyl, Di-$C_1$-$C_4$-alkylaminocarbonyl, Phenylaminocarbonyl, das unsubstituiert oder am Phenyl gleich oder verschieden bis zu dreifach

durch Halogen, $C_1$-$C_4$-Alkyl, Halogen-$C_1$-$C_4$-alkyl, Halogen-$C_1$-$C_4$- alkoxy oder $C_1$-$C_4$-Alkoxy oder einfach durch Cyano oder Nitro substituiert ist; Dioxolan-2-yl, das unsubstituiert ist oder durch ein oder zwei $C_1$-$C_4$-Alkylreste substituiert ist; oder Dioxan-2-yl, das unsubstituiert ist oder durch ein oder zwei $C_1$-$C_4$-Alkylreste substituiert ist;

$C_1$-$C_4$-Alkyl, das durch Cyano, Nitro, Carboxyl, $C_1$-$C_8$-Alkylthio-$C_2$-$C_8$-alkoxycarbonyl, $C_1$-$C_8$-Aikoxy-$C_2$-$C_8$-alkoxycarbonyl substituiert ist;

X Sauerstoff oder Schwefel;

X′ Sauerstoff oder Schwefel;

$R_5$ Wasserstoff, $C_1$-$C_{10}$-Alkyl; $C_1$-$C_4$-Alkoxy-$C_1$-$C_4$-alkyl; Halogen-$C_1$-$C_8$-alkyl; $C_1$-$C_{10}$-Alkylthio-$C_1$-$C_4$-alkyl; Di-$C_1$-$C_4$-alkylamino-$C_1$-$C_4$-alkyl; Cyano-$C_1$-$C_8$-alkyl; $C_3$-$C_8$-Alkenyl; Halogen-$C_2$-$C_8$-alkenyl; $C_3$-$C_8$-Alkinyl; $C_3$-$C_7$-Cycloalkyl; $C_3$-$C_7$-Cycloalkyl-$C_1$-$C_4$-alkyl; Halogen-$C_3$-$C_7$-cycloalkyl; Benzyl, das unsubstituiert oder am Phenylring gleich oder verschieden bis zu dreifach durch Halogen, $C_1$-$C_4$-Alkyl; Halogen-$C_1$-$C_4$-alkyl, Halogen-$C_1$-$C_4$-alkoxy oder $C_1$-$C_4$-alkoxy substituiert ist; Alkali-, Erdalkali-Ionen und Ammoniumionen; oder die Gruppe $-[CHR_6(CH_2)_n]-COOR_7$;

$R_6$ Wasserstoff; $C_1$-$C_4$-Alkyl;

$R_7$ Wasserstoff; $C_1$-$C_6$-Alkyl; $C_3$-$C_8$-Alkenyl; $C_3$-$C_8$-Alkinyl; $C_1$-$C_8$-Alkoxy-$C_2$-$C_8$-alkyl; $C_1$-$C_8$-Alkylthio-$C_2$-$C_8$-alkyl; $C_3$-$C_7$-Cycloalkyl;

$R_8$ Wasserstoff; $C_1$-$C_4$-Alkyl; Halogen-$C_1$-$C_4$-alkyl; $C_1$-$C_4$-Alkoxy-$C_1$-$C_4$-alkyl;

$R_9$ und $R_{10}$ unabhängig voneinander jeweils $C_1$-$C_4$-Alkyl; $C_2$-$C_4$-Halogenalkyl oder $C_2$-$C_8$-Alkoxyalkyl, oder

$R_9$ und $R_{10}$ zusammen eine Aethylen- oder Propylenbrücke oder einen 1,2-Cyclohexanylenkörper, wobei diese Gruppen unsubstituiert sind oder durch ein bis zwei Reste aus der Gruppe $C_1$-$C_4$-Alkyl, $C_1$-$C_4$-Halogenalkyl oder $C_1$-$C_4$-Hydroxyalkyl substituiert sind;

$R_{11}$ H, $C_1$-$C_5$-Alkyl, $C_3$-$C_7$-Alkenyl;

$R_{12}$ unabhängig voneinander H, $C_1$-$C_8$-Alkyl und

n 0,1,2,3 oder 4

bedeutet unter Einschluss der Salze oder Komplexe der Verbindungen der Formel I mit Säuren, Basen oder Komplexbildnern, sowie der stereoisomeren Verbindungen der Verbindungen der Formel I.

2. 1-Phenyl-piperidin-2,6-dione gemäss Anspruch 1, der Formel Ia

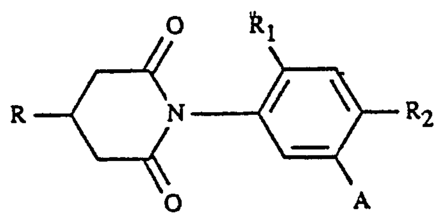 (Ia),

worin

R Wasserstoff; oder $C_1$-$C_6$-Alkyl; Halogen-$C_1$-$C_7$-alkyl;

$R_1$ Wasserstoff; oder Halogen;

$R_2$ Wasserstoff; Cyano; Nitro; oder Halogen;

A Wasserstoff; Cyano; Nitro; $COR^3$; oder $X-R_4$;

$R_3$ OH; Halogen; oder $X'-R^5$;

$R_4$ Wasserstoff; $C_1$-$C_6$-Alkyl; $C_3$-$C_6$-Alkenyl; oder $C_3$-$C_6$-Alkinyl;

X Sauerstoff oder Schwefel;

X′ Sauerstoff oder Schwefel;

$R_5$ Wasserstoff; $C_1$-$C_6$-Alkyl; oder die Gruppe $-[CHR_6(CH_2)_n]-COOR_7$;

$R_6$ Wasserstoff oder $C_1$-$C_4$-Alkyl;

$R_7$ Wasserstoff oder $C_1$-$C_6$-Alkyl; und

n die Zahl 0,1,2,3 oder 4;

bedeutet unter Einschluss der Salze oder Komplexe der Verbindungen dieser Formel mit Säuren, Basen oder Komplexbildnern.

3. 1-Phenyl-piperidin-2,6-dione gemäss Anspruch 1, der Formel Ib

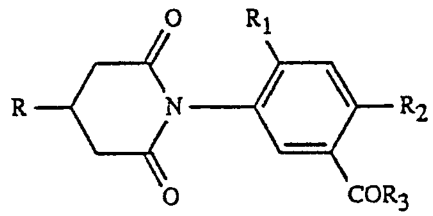

(Ib)

worin R, $R_1$, $R_2$ und $R_3$ wie im Anspruch 1 definiert sind.

4. 1-Phenyl-piperidin-2,6-dione gemäss Anspruch 1, der Formel Ic

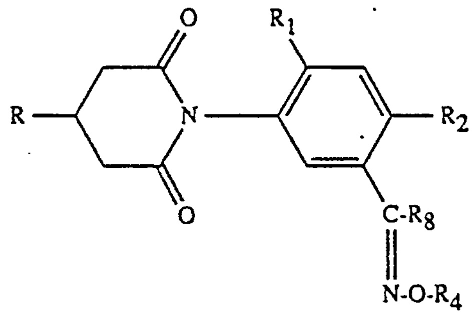

(Ic),

worin R, $R_1$, $R_2$, $R_8$ und $R_4$ die im Anspruch 1 gegebene Bedeutung haben.

5. 1-Phenyl-piperidin-2,6-dione gemäss Anspruch 1, der Formel Id

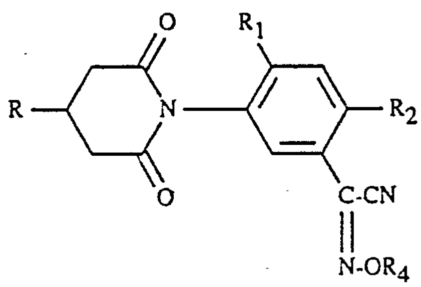

(Id),

worin R, $R_1$, $R_2$ und $R_4$ die im Anspruch 1 gegebene Bedeutung haben.

6. 1-Phenyl-piperidin-2,6-dione gemäss Anspruch 1, der Formel Ie

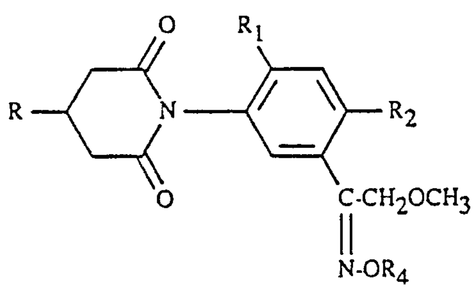

(Ie),

worin R, $R_1$, $R_2$ und $R_4$ die im Anspruch 1 gegebene Bedeutung haben.

7. 1-Phenyl-piperidin-2,6-dione gemäss Anspruch 1, der Formel If

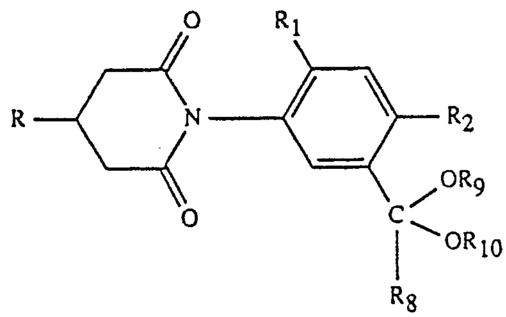

(If),

worin R, $R_1$, $R_2$, $R_8$, $R_9$ und $R_{10}$ die im Anspruch 1 gegebene Bedeutung haben.

8. 1-Phenyl-piperidin-2,6-dione gemäss Anspruch 1, der Formel Ig

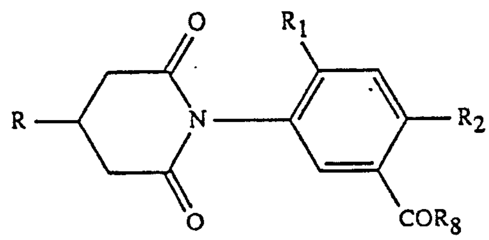

(Ig),

worin R, $R_1$, $R_2$, und $R_3$ die im Anspruch 1 gegebene Bedeutung haben.

9. 1-Phenyl-piperidin-2,6-dione gemäss Anspruch 1, der Formel Ih

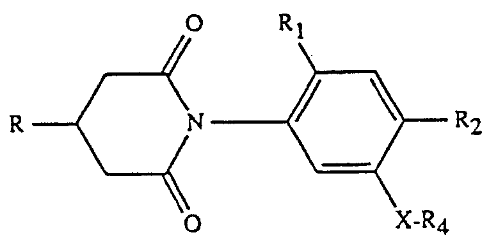

(Ih),

worin R, $R_1$, $R_2$, $R_4$ und X die im Anspruch 1 gegebene Bedeutung haben.

10. 1-Phenyl-piperidin-2,6-dione gemäss Anspruch 1, der Formel Ii

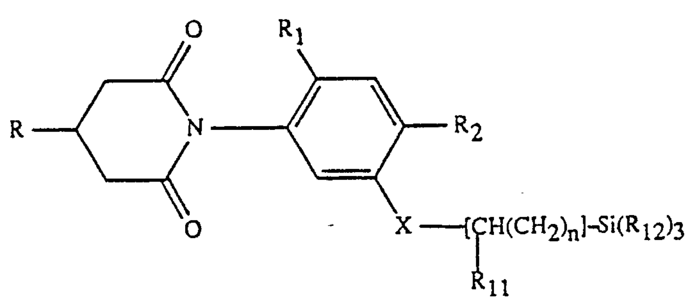

(Ii),

worin n, R, $R_1$, $R_2$, $R_{11}$ und $R_{12}$ die im Anspruch 1 gegebene Bedeutung haben.

11. 1-Phenyl-piperidin-2,6-dione gemäss Anspruch 1, der Formel Ij

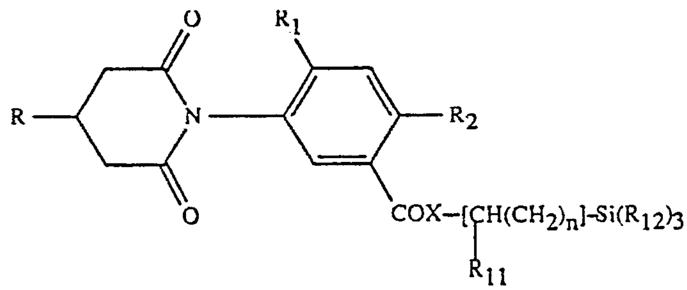

(Ij),

worin n, $R_1$, $R_2$, $R_{11}$, $R_{12}$ und X die im Anspruch 1 gegebene Bedeutung haben.

12. 1-Phenyl-piperidin-2,6-dione gemäss Anspruch 1, der Formel Ik

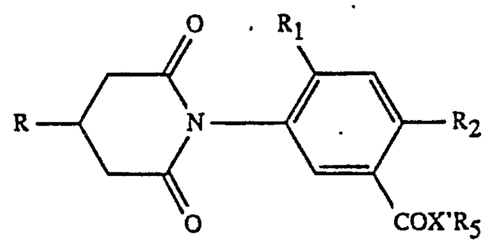

(Ik)

worin die Reste R, $R_1$, $R_2$, $R_5$ und X′ die im Anspruch 1 gegebene Bedeutung haben.

13. 1-Phenyl-piperidin-2,6-dione gemäss Anspruch 1,
worin
$R_1$ Fluor und
$R_2$ Chlor bedeutet und die Reste R und A wie im Anspruch 1 definiert sind.

14. 1-Phenyl-piperidin-2,6-dione gemäss Anspruch 1,
worin A Isopropyloxy oder Propargyloxy bedeutet und die Reste R, $R_1$ und $R_2$ wie im Anspruch 1 definiert sind.

15. 1-Phenyl-piperidin-2,6-dione gemäss Anspruch 1,
worin
A $-COX'-[CHR_6-(CH_2)_n]-COOR_7$,
X′ Sauerstoff oder Schwefel,
$R_6$ Wasserstoff oder Methyl,
n 0 oder 1,
$R_7$ $C_1$-$C_4$-Alkyl bedeutet und die Reste R, $R_1$ und $R_2$ wie im Anspruch 1 definiert sind.

16. Verfahren zur Herstellung von 1-Phenyl-piperidin-2,6-dionen der Formel I gemäss Anspruch 1, gekennzeichnet durch die Umsetzung von Glutarsäureanhydriden der Formel II

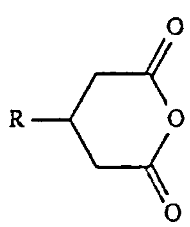

(II),

worin R die im Anspruch 1 gegebene Bedeutung hat mit einem Anilin der Formel III

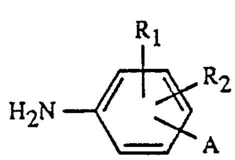

(III),

worin A, $R_1$ und $R_2$ die im Anspruch 1 gegebene Bedeutung hat, zum Glutarsäuremonoanilid der Formel IV kondensiert

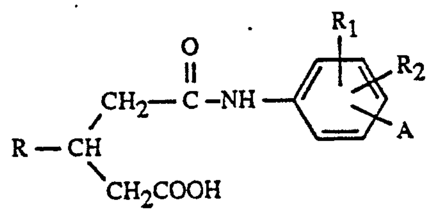

(IV),

worin A, R, $R_1$ und $R_2$ die im Anspruch 1 gegebene Bedeutung haben und anschliessend mit einem Kondensationsmittel zum 1-Phenylpiperidin-2,6-dione der Formel I cyclisiert,

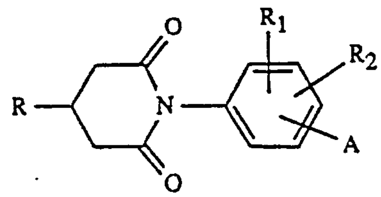

(I),

worin A, $R_1$ und $R_2$ die im Anspruch 1 gegebene Bedeutung haben.

17. Herbizides Mittel dadurch gekennzeichnet, dass es neben inerten Träger- und Zusatzstoffen und einem Tensid als Wirkstoff ein 1-Phenylpiperidin-2,6-dion gemäss Anspruch 1 enthält.

18. Verfahren zur Bekämpfung unerwünschten Pflanzenwuchses, dadurch gekennzeichnet, dass man auf die zu bekämpfende Pflanze oder deren Standfläche eine herbizid wirksame Menge einer Verbindung der Formel I gemäss Anspruch 1 einwirken lässt.

19. Verfahren gemäss Anspruch 18 zur pre- oder postemergenten Bekämpfung von Unkräutern in Nutzpflanzenkulturen.

20. Verfahren gemäss Anspruch 18 zur Bekämpfung von Unkräutern in Getreide, Mais, Hirse, Reis, Baumwolle, Soja oder Sonnenblumen.

21. Verbindungen der Formel IV

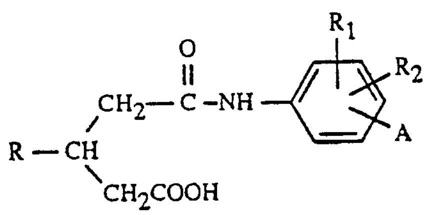

(IV),

worin die Reste R, $R_1$, $R_2$ und A wie im Anspruch 1 definiert sind.

22. Verfahren zur Herstellung von Verbindungen der Formel IV gemäss Anspruch 21, dadurch gekennzeichnet, dass man ein Glutarsäureanhydrid der Formel II mit einem Anilin der Formel III umsetzt

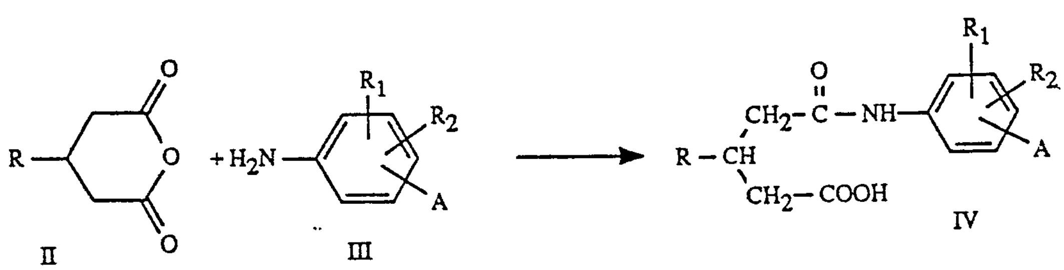

II III IV

worin R, $R_1$, $R_2$ und A die im Anspruch 1 gegebene Bedeutung haben.

Europäisches Patentamt

# EUROPÄISCHER RECHERCHENBERICHT

Nummer der Anmeldung

EP 90810246.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| X | CHEMICAL ABSTRACTS, Band 106, Nr. 11, 16. März 1987, Columbus, Ohio, USA<br>T.KOMETANI et al. "Asynthesis of succinimides and glutarimides from cyclic anhydrides"<br>Seite 584, Spalte 2, Zusammenfassung 84 339k<br>& Tetrahedron Lett., 27(8), 919-22 (1986)<br>-- | 1,2,16 | C 07 D 211/88<br>C 07 D 405/12<br>C 07 D 409/12<br>C 07 C 235/74<br>A 01 N 43/40 |
| A | GB - A - 1 593 809<br>(CIBA-GEIGY)<br>* Ansprüche 1,24,30 *<br>-- | 1,16, 17 | |
| A | CH - A - 504 412<br>(STERLIN DRUG)<br>* Anspruch 1; Spalte 4, Zeilen 20-62 *<br>---- | 1,16, 21,22 | |
| | | | RECHERCHIERTE SACHGEBIETE (Int Cl 5) |
| | | | C 07 D 211/00<br>C 07 D 405/00<br>C 07 D 409/00<br>C 07 C 235/00 |

Der vorliegende Recherchenbericht wurde für alle Patentansprüche erstellt.

| Recherchenort | Abschlußdatum der Recherche | Prüfer |
|---|---|---|
| WIEN | 29-06-1990 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument